# EUROPEAN PATENT APPLICATION

(11) **EP 4 501 914 A1**
(43) Date of publication of application: **05.02.2025**
(21) Application number: 23779833.5
(22) Date of filing: 20.03.2023
(51) Int. Cl.: C07D 233/60, C07C 51/41, C07C 53/10, C07C 53/122, C07C 57/04, C08B 15/08, C08B 16/00, C08K 5/17, C08L 1/02

(54) **ONIUM SALT, ONIUM SALT COMPOSITION, LIQUID COMPOSITION CONTAINING ONIUM SALT AND POLYSACCHARIDE AND PRODUCTION METHOD THEREOF, AND METHOD FOR RECOVERING POLYSACCHARIDE**

(30) Priority: 31.03.2022 JP 2022058919; 29.09.2022 JP 2022157135
(71) Applicant: Koei Chemical Company, Limited, Sodegaura-shi, Chiba 299-0266 (JP)
(72) Inventor: TAKECHI, Yoshiaki, Sodegaura-shi, Chiba 299-0266 (JP); TAMAKOSHI, Satomi, Sodegaura-shi, Chiba 299-0266 (JP); KOBAYASHI, Yumi, Sodegaura-shi, Chiba 299-0266 (JP)
(74) Representative: Müller-Boré & Partner Patentanwälte PartG mbB
(86) International application number: PCT/JP2023/010873
(87) International publication number: WO 2023/189817

(57) **Abstract**

The present invention relates to an onium salt, a liquid composition comprising the onium salt and polysaccharides, and a polysaccharide recovery method. According to the present invention, it is possible to provide an onium salt with very high polysaccharide solubility at 10 to 50°C, particularly at a temperature around room temperature (25°C). It is also possible to provide a liquid composition comprising the onium salt and polysaccharides as a composition suitable for recovering polysaccharides, and a method for recovering polysaccharides efficiently using such a liquid composition comprising the onium salt and polysaccharides.

## Description

### Technical Field

The present invention relates to an onium salt, an onium salt composition, a liquid composition comprising the onium salt and polysaccharides and a production method therefor, and a polysaccharide recovery method.

### Background Art

Polysaccharides are substances composed of a large number of monosaccharide molecules polymerized by glycosidic bonds. Among these, cellulose, which is a polysaccharide composed of β-glucose molecules, is the most abundant biomass resource on earth, and research into its dissolution and molding as an environmentally friendly material has been widely conducted. Furthermore, chitin and chitosan, which are polysaccharides whose main sugar components are N-acetylglucosamine and glucosamine, are obtained from crustacean shells and are used in various technical fields as materials with excellent biocompatibility and biodegradability. However, these polysaccharides have strong hydrogen bonds between or within molecular chains, making them difficult to dissolve in common solvents.

Onium salts have been proposed as solvents for dissolving cellulose. PTL 1 discloses 1-butyl-3-methylimidazolium chloride etc., and NPL 1 discloses 1-ethyl-3-methylimidazolium acetate etc.

For the purpose of dissolving various polysaccharides, such as cellulose, at around room temperature, PTL 2 discloses 1-ethyl-3-methylimidazolium dimethyl phosphate etc. Further, PTL 3 discloses N,N-diethyl-N-methyl-N-(2-methoxyethyl)ammonium 3-(2-methoxyethoxy)propanoate etc. as onium salts for dissolving cellulose.

Onium salts have also been proposed as solvents for dissolving chitin. NPL 2 discloses 1-butyl-3-methylimidazolium acetate etc.

### Citation List

### Patent Literature

PTL 1: JP2005-506401A
PTL 2: JP2010-132558A
PTL 3: WO2014/087646

### Non-patent Literature

NPL 1: Green Chemistry, 2009, vol. 11, pp. 417-424.
NPL 2: Polymer, 2008, vol. 49, pp. 2321-2327.

### Summary of Invention

### Technical Problem

The prior art has proposed various onium salts; however, most onium salts have cellulose solubility as low as 5% or less at around 25°C. Therefore, to use these onium salts as solvents for dissolving cellulose, it was necessary to heat them. For chitin, sufficient solubility cannot be obtained unless onium salts are heated to about 100°C. An object of the present invention is to provide an onium salt with high polysaccharide solubility at 10 to 50°C, particularly at a temperature around room temperature (25°C). Further, an object of the present invention is to provide a liquid composition comprising the onium salt and polysaccharides as a composition suitable for recovering polysaccharides, and a method for recovering polysaccharides efficiently using such a liquid composition comprising the onium salt and polysaccharides.

### Solution to Problem

The present inventors conducted extensive research on substances that can be used as solvents for sufficiently dissolving polysaccharides at 10 to 50°C, particularly at a temperature around room temperature (25°C). The present invention has thus been completed.

More specifically, the present invention provides the following [1] to [20].
[1] An onium salt represented by the following Formula (1): 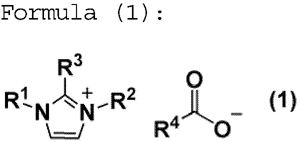
   wherein R¹ and R² are the same or different, and each represents a C₂-C₁₂ hydrocarbon group containing 1 to 5 heteroatoms or a group represented by the following Formula (1a):

      -O-R^{a} (1a)
   wherein R^{a} represents a C₁-C₁₂ hydrocarbon group that may contain 1 to 4 heteroatoms;
   R³ represents a hydrogen atom or a hydrocarbon group that may contain a heteroatom, wherein the heteroatom is at least one member selected from the group consisting of nitrogen, oxygen, and sulfur atoms; and
   R⁴ represents a hydrocarbon group, provided that when R¹ and R² are methoxyethyl groups and R³ is a hydrogen atom, R⁴ represents a C₂ or more hydrocarbon group.
[2] The onium salt according to [1], wherein R³ in Formula (1) is a hydrogen atom or a methyl group.
[3] The onium salt according to [1] or [2], wherein R¹ and R² in Formula (1) are the same or different, and each is represented by Formula (2):
   Formula (2):

   -R⁵-X-R⁶ (2)

   wherein R⁵ represents a C₂-C₅ divalent hydrocarbon group, R⁶ represents a C₁-C₁₀ hydrocarbon group that may contain 1 to 4 heteroatoms, the total number of carbon atoms in R⁵ and R⁶ is 3 to 12, the heteroatom is at least one member selected from the group consisting of nitrogen, oxygen, and sulfur atoms, and X represents an oxygen atom or a sulfur atom.
[4] The onium salt according to [3], wherein in Formula (2), R⁵ is a propane-1,3-diyl group, R⁶ is a C₁-C₉ hydrocarbon group that may contain 1 to 4 heteroatoms, and X is an oxygen atom.
[5] The onium salt according to any one of [1] to [4], wherein R¹ and R² in Formula (1) are the same.
[6] The onium salt according to any one of [1] to [5], wherein R⁴ in Formula (1) is a methyl group, CH₂=CH-, or CH₂=C(CH₃)-.
[7] An onium salt composition comprising two or more onium salts represented by the following Formula (1): 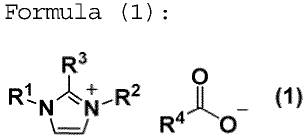
   wherein R¹ and R² are the same or different, and each represents a C₂-C₁₂ hydrocarbon group containing 1 to 5 heteroatoms or a group represented by the following Formula (1a):

      -O-R^{a} (1a)
   wherein R^{a} represents a C₁-C₁₂ hydrocarbon group that may contain 1 to 4 heteroatoms;
   R³ represents a hydrogen atom or a hydrocarbon group that may contain a heteroatom, wherein the heteroatom is at least one member selected from the group consisting of nitrogen, oxygen, and sulfur atoms; and
   R⁴ represents a hydrocarbon group.
[8] An onium hydroxide salt represented by the following Formula (3): 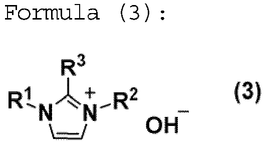
   wherein R¹ and R² are the same or different, and each represents a C₂-C₁₂ hydrocarbon group containing 1 to 5 heteroatoms or a group represented by the following Formula (1a):

      -O-R^{a} (1a)
   wherein R^{a} represents a C₁-C₁₂ hydrocarbon group that may contain 1 to 4 heteroatoms; and
   R³ represents a hydrogen atom or a hydrocarbon group that may contain a heteroatom, wherein the heteroatom is at least one member selected from the group consisting of nitrogen, oxygen, and sulfur atoms,
   provided that a case where R¹ and R² are methoxyethyl groups and R³ is a hydrogen atom is excluded.
[9] A method for producing an onium salt represented by Formula (1), comprising reacting glyoxal, an amine compound represented by Formula (6a), an amine compound represented by Formula (6b), an aldehyde compound represented by Formula (7), and a carboxylic acid represented by Formula (4):
   Formula (6a):

   R¹-NH₂ (6a)

   Formula (6b):

   R²-NH₂ (6b)

   wherein in Formulas (6a) and (6b), R¹ and R² are the same or different, and each represents a C₂-C₁₂ hydrocarbon group containing 1 to 5 heteroatoms or a group represented by the following Formula (1a);

      -O-R^{a} (1a)
   wherein R^{a} represents a C₁-C₁₂ hydrocarbon group that may contain 1 to 4 heteroatoms;
      Formula (7):

      R³-CHO (7)
   wherein R³ represents a hydrogen atom or a hydrocarbon group that may contain a heteroatom, wherein the heteroatom is at least one member selected from the group consisting of nitrogen, oxygen, and sulfur atoms;
      Formula (4):

      R⁴-COOH (4)
   wherein R⁴ represents a hydrocarbon group; and
      Formula (1): 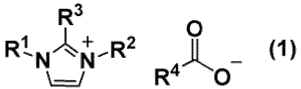
   wherein R¹ to R⁴ are as defined above.
[10] A method for producing a compound represented by Formula (1), comprising exchanging ions in a compound represented by Formula (3) using a compound represented by Formula (4): 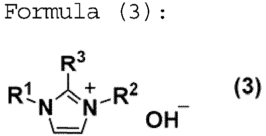
   wherein R¹ and R² are the same or different, and each represents a C₂-C₁₂ hydrocarbon group containing 1 to 5 heteroatoms or a group represented by the following Formula (1a):

      -O-R^{a} (1a)
   wherein R^{a} represents a C₁-C₁₂ hydrocarbon group that may contain 1 to 4 heteroatoms;
   R³ represents a hydrogen atom or a hydrocarbon group that may contain a heteroatom, wherein the heteroatom is at least one member selected from the group consisting of nitrogen, oxygen, and sulfur atoms;
      Formula (4):

      R⁴-COOH (4)
   wherein R⁴ represents a hydrocarbon group; and
      Formula (1): 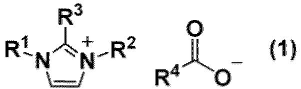
   wherein R¹ to R⁴ are as defined above.
[11] A polysaccharide-containing liquid composition comprising at least one polysaccharide and an onium salt represented by the following Formula (1) or the onium salt composition according to [7], wherein the polysaccharide is dissolved in the onium salt: 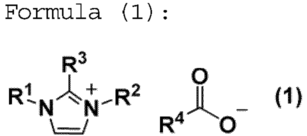
   wherein R¹ and R² are the same or different, and each represents a C₂-C₁₂ hydrocarbon group containing 1 to 5 heteroatoms or a group represented by the following Formula (1a):

      -O-R^{a} (1a)
   wherein R^{a} represents a C₁-C₁₂ hydrocarbon group that may contain 1 to 4 heteroatoms;
   R³ represents a hydrogen atom or a hydrocarbon group that may contain a heteroatom, wherein the heteroatom is at least one member selected from the group consisting of nitrogen, oxygen, and sulfur atoms; and
   R⁴ represents a hydrocarbon group, provided that when R¹ is (2-methoxyethoxy)methyl, R² is 2-[2-(2-methoxyethoxy)ethoxy]ethyl, and R³ is a hydrogen atom, R⁴ represents a C₂ or more hydrocarbon group.
[12] The polysaccharide-containing liquid composition according to [11], further comprising at least one organic solvent, wherein the polysaccharide is dissolved in a medium containing the onium salt or onium salt composition and the organic solvent.
[13] The polysaccharide-containing liquid composition according to [12], wherein the organic solvent is at least one organic solvent selected from the group consisting of dimethyl sulfoxide, N,N-dimethylacetamide, N,N-dimethylformamide, and N-methyl-2-pyrrolidone.
[14] The polysaccharide-containing liquid composition according to [11], wherein the polysaccharide is cellulose.
[15] A method for producing the polysaccharide-containing liquid composition according to any one of [11] to [14], comprising dissolving a polysaccharide using an onium salt represented by the following Formula (1) or the onium salt composition according to [7]: 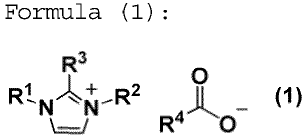
   wherein R¹ and R² are the same or different, and each represents a C₂-C₁₂ hydrocarbon group containing 1 to 5 heteroatoms or a group represented by the following Formula (1a):

      -O-R^{a} (1a)
   wherein R^{a} represents a C₁-C₁₂ hydrocarbon group that may contain 1 to 4 heteroatoms;
   R³ represents a hydrogen atom or a hydrocarbon group that may contain a heteroatom, wherein the heteroatom is at least one member selected from the group consisting of nitrogen, oxygen, and sulfur atoms; and
   R⁴ represents a hydrocarbon group.
[16] The method for producing the polysaccharide-containing liquid composition according to [15], wherein the temperature to dissolve the polysaccharide is 50°C or less.
[17] The method for producing the polysaccharide-containing liquid composition according to [15] or [16], wherein the polysaccharide is cellulose.
[18] A polysaccharide recovery method, comprising bringing the polysaccharide-containing liquid composition according to any one of [11] to [14] into contact with at least one poor solvent.
[19] The polysaccharide recovery method according to [18], wherein the poor solvent is selected from the group consisting of water, methanol, ethanol, acetone, and a mixed solvent thereof.
[20] The polysaccharide recovery method according to [18] or [19], wherein the polysaccharide is cellulose.

### Advantageous Effects of Invention

According to the present invention, it is possible to provide an onium salt with very high polysaccharide solubility at 10 to 50°C, particularly at a temperature around room temperature (25°C). It is also possible to provide a liquid composition comprising the onium salt and polysaccharides as a composition suitable for recovering polysaccharides. Further, it is possible to provide a method for recovering polysaccharides efficiently using such a liquid composition comprising the onium salt and polysaccharides.

### Description of Embodiments

The present invention is described in detail below. The present invention relates to an onium salt represented by the following Formula (1) (hereinafter referred to as the onium salt (1)). 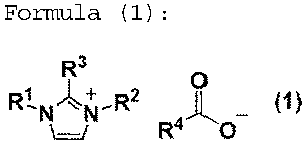
wherein R¹ and R² are the same or different, and each represents a C₂-C₁₂ hydrocarbon group containing 1 to 5 heteroatoms or a group represented by the following Formula (1a):

   -O-R^{a} (1a)
wherein R^{a} represents a C₁-C₁₂ hydrocarbon group that may contain 1 to 4 heteroatoms;
R³ represents a hydrogen atom or a hydrocarbon group that may contain a heteroatom, wherein the heteroatom is at least one member selected from the group consisting of nitrogen, oxygen, and sulfur atoms; and
R⁴ represents a hydrocarbon group.

In Formula (1), the "C₂-C₁₂ hydrocarbon group containing 1 to 5 heteroatoms" in R¹ and R² may be linear, branched, or cyclic, or may contain a double bond or a triple bond. The number of heteroatoms is preferably 1 to 3, and more preferably 1 or 2. The number of carbon atoms in the hydrocarbon group is preferably 3 to 10, and more preferably 4 to 7.

In Formula (1), examples of the "heteroatom" in R¹ and R² include nitrogen, oxygen, and sulfur atoms.

In Formula (1), the "C₂-C₁₂ hydrocarbon group containing 1 to 5 heteroatoms" in R¹ and R² is a heteroatom-free C₂-C₁₂ hydrocarbon group that is interrupted or replaced by a structure, such as -O-, -N<, -NH-, -S-, -C(=O)-O-, -C(=O)-, =N-, -C(=O)-N<, -C(=O)-NH-, -O-C(=O)-O-, -O-C(=O) -N<, -O-C(=O)-NH-, -C(=O)-N[-C(=O)-]-, -C(=O)-NH-C(=O)-, >N-C(=O)-N<, >N-C(=O)-NH-, -HN-C(=O)-NH-, -S(=O)-, -S(=O)₂-, -S(=O)₂-O-, -O-S(=O)₂-O-, >N-C(=S)-N<, >N-C(=S)-NH-, -HN-C(=S)-NH-, -C(=N-)-, or -C(=NH)-. The number or combination of interruptions or replacements by such structures is not particularly limited as long as the number of heteroatoms in R¹ and R² is 1 to 5.

Preferred is a hydrocarbon group that is interrupted or replaced by the structure -O- or -S-, and more preferred is a hydrocarbon group that is interrupted or replaced by the structure -O-.

In the present invention, the structure of the hydrocarbon group interrupted or replaced by the structure -O- or -S- may be an ether structure or a thioether structure, and may also be, for example, an epoxy structure or a thioepoxy structure, such as an epoxy group, a thioepoxy group, a glycidyl ether group, or an epoxycyclohexyl group. In this case, R¹ or R² may be an epoxy group, a thioepoxy group, or the like.

When R¹ and R² include two or more structures -O-or/and -S-, the number of carbon atoms between the structures -O-or/and -S- is desirably 2 or more.

It is more desirable that the number of carbon atoms between the nitrogen atom on the imidazole ring to which each of R¹ and R² is bonded and the heteroatom in R¹ or R² that is closest to the nitrogen atom on the imidazole ring is 2 or more.

In Formula (1), the "heteroatom-free C₂-C₁₂ hydrocarbon group" in R¹ and R² may be linear, branched, or cyclic, or may contain a double bond or a triple bond. Examples of the heteroatom-free C₂-C₁₂ hydrocarbon group include alkyl, alkenyl, alkynyl, cycloalkyl, cycloalkenyl, aryl, and aralkyl groups, but are not particularly limited thereto.

In Formula (1), examples of the "C₂-C₁₂ hydrocarbon group containing 1 to 5 heteroatoms" in R¹ and R² include a C₂-C₁₂ linear or branched alkyl group containing 1 to 5 heteroatoms, a C₂-C₁₂ linear or branched alkenyl group containing 1 to 5 heteroatoms, a C₂-C₁₂ linear or branched alkynyl group containing 1 to 5 heteroatoms, a C₃-C₁₂ optionally branched cycloalkyl group containing 1 to 5 heteroatoms, a C₃-C₁₂ optionally branched cycloalkenyl group containing 1 to 5 heteroatoms, a C₆-C₁₂ optionally branched aryl group containing 1 to 5 heteroatoms, a C₇-C₁₂ optionally branched aralkyl group containing 1 to 5 heteroatoms, and the like. Preferred are a C₂-C₁₂ linear or branched alkyl group containing 1 to 5 heteroatoms, a C₂-C₁₂ linear or branched alkenyl group containing 1 to 5 heteroatoms, and a C₃-C₁₂ optionally branched cycloalkyl group containing 1 to 5 heteroatoms.

The "C₂-C₁₂ linear or branched alkyl group containing 1 to 5 heteroatoms" is preferably a C₃-C₁₀ linear or branched alkyl group containing 1 to 3 heteroatoms. In another embodiment, preferred is a C₃-C₁₀ linear or branched alkyl group containing 1 or 2 heteroatoms, and more preferred is a C₄-C₇ linear or branched alkyl group containing 1 or 2 heteroatoms.

The "C₂-C₁₂ linear or branched alkenyl group containing 1 to 5 heteroatoms" is preferably a C₃-C₁₀ linear or branched alkenyl group containing 1 to 3 heteroatoms. In another embodiment, preferred is a C₃-C₁₀ linear or branched alkenyl group containing 1 or 2 heteroatoms, and more preferred is a C₄-C₇ linear or branched alkenyl group containing 1 or 2 heteroatoms.

The "C₃-C₁₂ optionally branched cycloalkyl group containing 1 to 5 heteroatoms" is preferably a C₃-C₁₀ optionally branched cycloalkyl group containing 1 to 3 heteroatoms. In another embodiment, preferred is a C₃-C₁₀ optionally branched cycloalkyl group containing 1 or 2 heteroatoms, and more preferred is a C₄-C₇ optionally branched cycloalkyl group containing 1 or 2 heteroatoms.

In Formula (1), specific examples of the heteroatom-free C₂-C₁₂ hydrocarbon groups in R¹ and R² include ethyl, propyl, isopropyl, butyl, sec-butyl, isobutyl, tert-butyl, pentyl, neopentyl, hexyl, octyl, 2-ethylhexyl, decyl, dodecyl, propenyl, isopropenyl, allyl, propargyl, butenyl, crotyl, butadienyl, cyclopropyl, cyclobutyl, cyclopentyl, cyclohexyl, norbornyl, adamantyl, phenyl, benzyl, tolyl, and 2,4,6-trimethylphenyl groups, but are not particularly limited thereto. When the heteroatom-free C₂-C₁₂ hydrocarbon group is interrupted or replaced by a structure, such as -O-, -N<, -NH-, -S-, -C(=O)-O-, -C(=O)-, =N-, -C(=O)-N<, -C(=O)-NH-, -O-C(=O)-O-, -O-C(=O)-N<, - O-C(=O)-NH-, -C(=O)-N[-C(=O)-]-, -C(=O)-NH-C(=O)-, >N-C(=O)-N<, >N-C(=O)-NH-, -HN-C(=O)-NH-, -S(=O)-, -S(=O)₂-, -S(=O)₂-O-, -O-S(=O)₂-O-, >N-C(=S)-N<, >N-C(=S)-NH-, -HN-C(=S)-NH-, -C(=N-)-, or -C(=NH)-, it becomes a "C₂-C₁₂ hydrocarbon group containing 1 to 5 heteroatoms."

In Formula (1a), R^{a} represents a C₁-C₁₂ hydrocarbon group that may contain 1 to 4 heteroatoms. The "C₁-C₁₂ hydrocarbon group that may contain 1 to 4 heteroatoms" may be linear, branched, or cyclic, or may contain a double bond or a triple bond.

In R^{a}, the hydrocarbon group may not contain a heteroatom or may contain 1 to 4 heteroatoms. When R^{a} does not contain a heteroatom, the number of carbon atoms in the hydrocarbon group is 1 to 12, preferably 1 to 6, and more preferably 1 to 3. When R^{a} contains a heteroatom, the number of heteroatoms is preferably 1 or 2. When R^{a} contains a heteroatom, the number of carbon atoms in the hydrocarbon group is 2 to 12, and preferably 2 to 6.

In Formula (1a), examples of the "heteroatom" when R^{a} is a C₂-C₁₂ hydrocarbon group containing 1 to 4 heteroatoms include nitrogen, oxygen, and sulfur atoms.

In Formula (1a), the "C₂-C₁₂ hydrocarbon group containing 1 to 4 heteroatoms" in R^{a} is a heteroatom-free C₂-C₁₂ hydrocarbon group that is interrupted or replaced by a structure, such as -O-, -N<, -NH-, -S-, -C(=O)-O-, -C(=O)-, =N-, -C(=O)-N<, -C(=O)-NH-, -O-C(=O)-O-, -O-C(=O) -N<, -O-C(=O)-NH-, -C(=O)-N[-C(=O)-]-, -C(=O)-NH-C(=O)-, >N-C(=O)-N<, >N-C(=O)-NH-, -HN-C(=O)-NH-, -S(=O)-, -S(=O)₂-, -S(=O)₂-O-, >N-C(=S)-N<, >N-C(=S)-NH-, - HN-C(=S)-NH-, -C(=N-)-, or -C(=NH)-. The number or combination of interruptions or replacements by such structures is not particularly limited as long as the number of heteroatoms in R^{a} is 1 to 4.

Preferred is a hydrocarbon group that is interrupted or replaced by the structure -O- or -S-, and more preferred is a hydrocarbon group that is interrupted or replaced by the structure -O-.

In the present invention, the structure of the hydrocarbon group interrupted or replaced by the structure -O- or -S- may be an ether structure or a thioether structure, and may also be, for example, an epoxy structure or a thioepoxy structure, such as an epoxy group, a thioepoxy group, a glycidyl ether group, or an epoxycyclohexyl group. In this case, R^{a} may be an epoxy group, a thioepoxy group, or the like.

In Formula (1a), when R^{a} includes two or more structures -O- or/and -S-, the number of carbon atoms between the structures -O- or/and -S- is desirably 2 or more. It is more desirable that the number of carbon atoms between the oxygen atom to which R^{a} is bonded and the heteroatom in R^{a} that is closest to the oxygen atom is 2 or more.

In Formula (1a), the "heteroatom-free C₁-C₁₂ hydrocarbon group" and "heteroatom-free C₂-C₁₂ hydrocarbon group" in R^{a} may be linear, branched, or cyclic, or may contain a double bond or a triple bond. Examples of the "heteroatom-free C₁-C₁₂ hydrocarbon group" and "heteroatom-free C₂-C₁₂ hydrocarbon group" include alkyl, alkenyl, alkynyl, cycloalkyl, cycloalkenyl, aryl, and aralkyl groups, but are not particularly limited thereto.

In Formula (1a), examples of the "C₁-C₁₂ hydrocarbon group that may contain 1 to 4 heteroatoms" in R^{a} include a C₁-C₁₂ linear or branched alkyl group that may contain 1 to 4 heteroatoms, a C₂-C₁₂ linear or branched alkenyl group that may contain 1 to 4 heteroatoms, a C₂-C₁₂ linear or branched alkynyl group that may contain 1 to 4 heteroatoms, a C₃-C₁₂ optionally branched cycloalkyl group that may contain 1 to 4 heteroatoms, a C₃-C₁₂ optionally branched cycloalkenyl group that may contain 1 to 4 heteroatoms, a C₆-C₁₂ optionally branched aryl group containing 1 to 5 heteroatoms, a C₇-C₁₂ optionally branched aralkyl group that may contain 1 to 4 heteroatoms, and the like. Preferred are a C₁-C₁₂ linear or branched alkyl group that may contain 1 to 4 heteroatoms, a C₂-C₁₂ linear or branched alkenyl group that may contain 1 to 4 heteroatoms, and a C₃-C₁₂ optionally branched cycloalkyl group that may contain 1 to 4 heteroatoms.

The "C₁-C₁₂ linear or branched alkyl group that may contain 1 to 4 heteroatoms" is preferably a C₁-C₆ linear or branched alkyl group that may contain 1 to 4 heteroatoms. In another embodiment, preferred is a C₂-C₁₂ linear or branched alkyl group that may contain 1 or 2 heteroatoms, and more preferred is a C₁-C₆ linear or branched alkyl group that may contain 1 or 2 heteroatoms. In still another embodiment, preferred is a C₁-C₁₂ linear or branched alkyl group, more preferred is a C₁-C₆ linear or branched alkyl group, and even more preferred is a C₁-C₃ linear or branched alkyl group.

The "C₂-C₁₂ linear or branched alkenyl group that may contain 1 to 4 heteroatoms" is preferably a C₂-C₆ linear or branched alkenyl group that may contain 1 to 4 heteroatoms. In another embodiment, preferred is a C₂-C₁₂ linear or branched alkenyl group that may contain 1 or 2 heteroatoms, and more preferred is a C₂-C₆ linear or branched alkenyl group that may contain 1 or 2 heteroatoms. In still another embodiment, preferred is a C₂-C₁₂ linear or branched alkenyl group, more preferred is a C₂-C₆ linear or branched alkenyl group, and even more preferred is a C₂ or C₃ linear or branched alkenyl group.

The "C₃-C₁₂ optionally branched cycloalkyl group that may contain 1 to 4 heteroatoms" is preferably a C₃-C₆ optionally branched cycloalkyl group that may contain 1 to 4 heteroatoms. In another embodiment, preferred is a C₃-C₁₂ optionally branched cycloalkyl group that may contain 1 or 2 heteroatoms, and more preferred is a C₃-C₆ optionally branched cycloalkyl group that may contain 1 or 2 heteroatoms. In still another embodiment, preferred is a C₃-C₁₂ optionally branched cycloalkyl group, more preferred is a C₃-C₆ optionally branched cycloalkyl group, and even more preferred is a C₃ optionally branched cycloalkyl group.

In Formula (1), when R^{a} is a heteroatom-free C₁-C₁₂ hydrocarbon group, specific examples of the "C₁-C₁₂ hydrocarbon group" include methyl, ethyl, propyl, isopropyl, butyl, sec-butyl, isobutyl, tert-butyl, pentyl, neopentyl, hexyl, octyl, 2-ethylhexyl, decyl, dodecyl, vinyl, ethynyl, propenyl, isopropenyl, allyl, propargyl, butenyl, crotyl, butadienyl, cyclopropyl, cyclobutyl, cyclopentyl, cyclohexyl, norbornyl, adamantyl, phenyl, benzyl, tolyl, and 2,4,6-trimethylphenyl groups, but are not particularly limited thereto.

In Formula (1), examples of the heteroatom-free C₂-C₁₂ hydrocarbon group in R^{a} include ethyl, propyl, isopropyl, butyl, sec-butyl, isobutyl, tert-butyl, pentyl, neopentyl, hexyl, octyl, 2-ethylhexyl, decyl, dodecyl, propenyl, isopropenyl, allyl, propargyl, butenyl, crotyl, butadienyl, cyclopropyl, cyclobutyl, cyclopentyl, cyclohexyl, norbornyl, adamantyl, phenyl, benzyl, tolyl, and 2,4,6-trimethylphenyl groups, but are not particularly limited thereto. When the heteroatom-free C₂-C₁₂ hydrocarbon group is interrupted or replaced by a structure, such as -O-, -N<, - NH-, -S-, -C(=O)-O-, -C(=O)-, =N-, -C(=O)-N<, -C(=O)-NH-, -O-C(=O)-O-, -O-C(=O)-N<, -O-C(=O)-NH-, -C(=O)-N[-C(=O)-]-, -C(=O)-NH-C(=O)-, >N-C(=O)-N<, >N-C(=O)-NH-, -HN-C(=O)-NH-, -S(=O)-, - S(=O)₂-, -S(=O)₂-O-, >N-C(=S)-N<, >N-C(=S)-NH-, -HN-C(=S)-NH-, - C(=N-)-, or -C(=NH)-, it becomes a "C₂-C₁₂ hydrocarbon group containing 1 to 4 heteroatoms."

Specific examples of the structures of R¹ and R² shown in Formula (1) include 2-methoxyethyl, 2-ethoxyethyl, 2-propoxyethyl, 2-isopropoxyethyl, 2-phenoxyethyl, 2-(benzyloxy)ethyl, 2-(p-methoxybenzyloxy)ethyl, 2-(2-methoxyethoxymethoxy) ethyl, 2-(2-benzyloxyethoxy)ethyl, 2-(dimethylamino)ethyl, 3-methoxypropyl, 3-ethoxypropyl, 3-propoxypropyl, 3-allyloxypropyl, 3-isopropoxypropyl, 3-(1-methylpropoxy) propyl, 3-(2-methylpropoxy)propyl, 3-(1,1-dimethylethoxy) propyl, 3-butoxypropyl, 3-pentyloxypropyl, 3-neopentyloxypropyl, 3-hexyloxypropyl, 3-cyclohexyloxypropyl, 3-heptyloxypropyl, 3-octyloxypropyl, 3-nonyloxypropyl, 3-(2-ethylhexan-1-yl)oxypropyl, 3-(2,4,6-trimethylphenoxy)propyl, 3-(2-methoxyethoxy)propyl, 3-(dimethylamino)propyl, 4-methoxybutyl, 4-ethoxybutyl, 4-propoxybutyl, 4-isopropoxybutyl, 4-allyloxybutyl, 4-butoxybutyl, 4-(1-methylpropoxy)butyl, 4-(2-methylpropoxy) butyl, 4-(1,1-dimethylethoxy)butyl, 4-pentyloxybutyl, 4-neopentyloxybutyl, 4-hexyloxybutyl, 4-cyclohexyloxybutyl, 4-heptyloxybutyl, 4-octyloxybutyl, 4-(2-ethylhexan-1-yl)oxybutyl, 5-methoxypentyl, 5-ethoxypentyl, 5-propoxypentyl, 5-isopropoxypentyl, 5-allyloxypentyl, 5-butoxypentyl, 5-(1-methylpropoxy)pentyl, 5-(2-methylpropoxy)pentyl, 5-(1,1-dimethylethoxy)pentyl, 5-pentyloxypentyl, 5-neopentyloxypentyl, 5-hexyloxypentyl, 5-cyclohexyloxypentyl, 5-heptyloxypentyl, 6-methoxyhexyl, 6-ethoxyhexyl, 6-propoxyhexyl, 6-isopropoxyhexyl, 6-allyloxyhexyl, 6-butoxyhexyl, 6-(1-methylpropoxy)hexyl, 6-(2-methylpropoxy)hexyl, 6-(1,1-dimethylethoxy)hexyl, 6-pentyloxyhexyl, 6-neopentyloxyhexyl, 6-hexyloxyhexyl, 6-cyclohexyloxyhexyl, (2-methoxycyclohexan-1-yl)methyl, (2-ethoxycyclohexan-1-yl)methyl, (2-propoxycyclohexan-1-yl)methyl, (2-isopropoxycyclohexan-1-yl)methyl, (2-butoxycyclohexan-1-yl)methyl, [2-(1-methylpropoxy)cyclohexan-1-yl]methyl, [2-(2-methylpropoxy)cyclohexan-1-yl]methyl, [2-(1,1-dimethylethoxy)cyclohexan-1-yl]methyl, (2-pentyloxycyclohexan-1-yl)methyl, (2-neopentyloxycyclohexan-1-yl)methyl, 2-methylthioethyl, 3-methylthiopropyl, 1-(methoxymethyl)propyl, (oxolan-2-yl)methyl, (furan-2-yl)methyl, 2-[(furan-2-yl)methylthio]ethyl, 2-(methylsulfonyl)ethyl, (thiophen-2-yl)methyl, p-methoxyphenyl, p-ethoxyphenyl, 3,4-methylenedioxyphenyl, 3,4-methylenedioxybenzyl, (7-oxabicyclo[2,2,1]heptan-2-yl)methyl, and the like; preferably 2-methoxyethyl, 3-methoxypropyl, 3-ethoxypropyl, 3-isopropoxypropyl, 3-butoxypropyl, 3-(2-methoxyethoxy)propyl, 3-methylthiopropyl, and 3-(dimethylamino)propyl; and more preferably 2-methoxyethyl, 3-methoxypropyl, and 3-(2-methoxyethoxy)propyl; however, the present invention is not limited thereto.

In Formula (1), it is preferable that R¹ and R² are the same.

In Formula (1), R³ represents a hydrogen atom or a hydrocarbon group that may contain a heteroatom. The "hydrocarbon group that may contain a heteroatom" may be linear, branched, or cyclic, or may contain a double bond or a triple bond. Further, the hydrocarbon group in R³ may not contain a heteroatom or may contain a heteroatom.

In Formula (1), when R³ is a heteroatom-free hydrocarbon group, the number of carbon atoms is preferably 1 to 12, more preferably 1 to 8, and even more preferably 1 to 3. When R³ is a heteroatom-containing hydrocarbon group, the number of carbon atoms is preferably 2 to 12, more preferably 2 to 8, and even more preferably 2 to 4.

In Formula (1), when R³ contains a heteroatom, the number of heteroatoms is preferably 1 to 5, and more preferably 1 or 2. The number of carbon atoms in the hydrocarbon group is 2 or more, preferably 2 to 12, more preferably 2 to 8, and even more preferably 2 to 4.

In Formula (1), when R³ is a heteroatom-containing hydrocarbon group, examples of the "heteroatom" include nitrogen, oxygen, and sulfur atoms.

When R³ is a heteroatom-containing hydrocarbon group, examples of the "heteroatom" include nitrogen, oxygen, and sulfur atoms. The number of heteroatoms is preferably 1 to 5, and more preferably 1 or 2.

In Formula (1), the "heteroatom-containing hydrocarbon group" in R³ is a heteroatom-free hydrocarbon group that is interrupted or replaced by a structure, such as -O-, -N<, -NH-, - S-, -C(=O)-O-, -C(=O)-, =N-, -C(=O)-N<, -C(=O)-NH-, -O-C(=O)-O-, -O-C(=O)-N<, -O-C(=O)-NH-, -C(=O)-N[-C(=O)-]-, -C(=O)-NH-C(=O)-, >N-C(=O)-N<, >N-C(=O)-NH-, -HN-C(=O)-NH-, -S(=O)-, -S(=O)₂-, - S(=O)₂-O-, -O-S(=O)₂-O-, >N-C(=S)-N<, >N-C(=S)-NH-, -HN-C(=S)-NH-, -C(=N-)-, or -C(=NH)-. The number or combination of interruptions or replacements by such structures is not particularly limited; however, the number of heteroatoms in R³ is preferably 1 to 5, and more preferably 1 or 2.

Preferred is a hydrocarbon group that is interrupted or replaced by the structure -O- or -S-, and more preferred is a hydrocarbon group that is interrupted or replaced by the structure -O-.

In the present invention, the structure of the hydrocarbon group interrupted or replaced by the structure -O- or -S- may be an ether structure or a thioether structure, and may also be, for example, an epoxy structure or a thioepoxy structure, such as an epoxy group, a thioepoxy group, a glycidyl ether group, or an epoxycyclohexyl group. In this case, R³ may be an epoxy group, a thioepoxy group, or the like.

When R³ includes two or more structures -O- or/and -S-, the number of carbon atoms between the structures -O- or/and -Sis desirably 2 or more.

In Formula (1), examples of the "hydrocarbon group that may contain a heteroatom" in R³ include a linear or branched alkyl group that may contain a heteroatom, a linear or branched alkenyl group that may contain a heteroatom, a linear or branched alkynyl group that may contain a heteroatom, an optionally branched cycloalkyl group that may contain a heteroatom, an optionally branched cycloalkenyl group that may contain a heteroatom, an optionally branched aryl group that may contain a heteroatom, and an optionally branched aralkyl group that may contain a heteroatom. Preferred are a linear or branched alkyl group that may contain a heteroatom, a linear or branched alkenyl group that may contain a heteroatom, and an optionally branched cycloalkyl group that may contain a heteroatom.

The "linear or branched alkyl group that may contain a heteroatom" is preferably a C₁-C₁₂ linear or branched alkyl group that may contain 1 to 5 heteroatoms. In another embodiment, preferred is a C₁-C₁₂ linear or branched alkyl group that may contain 1 or 2 heteroatoms, more preferred is a C₁-C₈ linear or branched alkyl group that may contain 1 or 2 heteroatoms, and even more preferred is a C₁-C₄ linear or branched alkyl group that may contain 1 or 2 heteroatoms. In still another embodiment, preferred is a C₁-C₁₂ linear or branched alkyl group, more preferred is a C₁-C₈ linear or branched alkyl group, and even more preferred is a C₁-C₃ linear or branched alkyl group.

The "linear or branched alkenyl group that may contain a heteroatom" is preferably a C₂-C₁₂ linear or branched alkenyl group that may contain 1 to 5 heteroatoms. In another embodiment, preferred is a C₂-C₁₂ linear or branched alkenyl group that may contain 1 or 2 heteroatoms, more preferred is a C₂-C₈ linear or branched alkenyl group that may contain 1 or 2 heteroatoms, and even more preferred is a C₂-C₄ linear or branched alkenyl group that may contain 1 or 2 heteroatoms. In still another embodiment, preferred is a C₂-C₁₂ linear or branched alkenyl group, more preferred is a C₂-C₈ linear or branched alkenyl group, and even more preferred is a C₂ or C₃ linear or branched alkenyl group.

The "optionally branched cycloalkyl group that may contain a heteroatom" is preferably a C₃-C₁₂ optionally branched cycloalkyl group that may contain 1 to 5 heteroatoms. In another embodiment, preferred is a C₃-C₁₂ optionally branched cycloalkyl group that may contain 1 or 2 heteroatoms, more preferred is a C₃-C₈ optionally branched cycloalkyl group that may contain 1 or 2 heteroatoms, and even more preferred is a C₃ or C₄ optionally branched cycloalkyl group that may contain 1 or 2 heteroatoms. In still another embodiment, preferred is a C₃-C₁₂ optionally branched cycloalkyl group, more preferred is a C₃-C₈ optionally branched cycloalkyl group, and even preferred is a C₃ cycloalkyl group.

In Formula (1), specific examples of the "heteroatom-free hydrocarbon group" in R³ include methyl, ethyl, propyl, isopropyl, butyl, sec-butyl, isobutyl, tert-butyl, pentyl, neopentyl, hexyl, octyl, 2-ethylhexyl, decyl, dodecyl, vinyl, ethynyl, propenyl, isopropenyl, allyl, propargyl, butenyl, crotyl, butadienyl, cyclopropyl, cyclobutyl, cyclopentyl, cyclohexyl, norbornyl, adamantyl, phenyl, benzyl, tolyl, styryl, and 2,4,6-trimethylphenyl groups; preferably methyl, ethyl, propyl, isopropyl, butyl, sec-butyl, isobutyl, tert-butyl, pentyl, neopentyl, and hexyl groups; and more preferably methyl, ethyl, propyl, isopropyl, and tert-butyl groups.

In Formula (1), the "heteroatom-containing hydrocarbon group" in R³ is preferably 3-indolyl, p-methoxyphenyl, 7-oxabicyclo[2,2,1]hepta-5-en-2-yl, 1,2-epoxyethyl, 1-methyl-2-methoxyvinyl, 2-furyl, 2-pyridyl, 4-imidazolyl, or benzyloxymethyl group; and more preferably 1-methyl-2-methoxyvinyl or 2-furyl group; however, the present invention is not limited thereto.

In Formula (1), R³ is preferably a hydrogen atom or a methyl group, and more preferably a hydrogen atom.

In Formula (1), some or all of R¹ to R³ may be bonded together to form a ring structure together with the nitrogen and carbon atoms to which they are bonded. Specific examples of the ring structure include, but are not particularly limited to, a cyclic structure in which R¹ and R², R¹ and R³, or R² and R³ are bonded together and bridged by a C₂-C₂₄ hydrocarbon containing 2 to 10 heteroatoms.

In Formula (1), R⁴ represents a hydrocarbon group, which may be linear, branched, or cyclic, or may contain a double bond or a triple bond. R⁴ is preferably a C₁-C₁₂ hydrocarbon group, more preferably a C₁-C₈ hydrocarbon group, and even more preferably a C₁-C₃ hydrocarbon group.

In Formula (1), examples of the "hydrocarbon group" in R⁴ include alkyl, alkenyl, alkynyl, cycloalkyl, cycloalkenyl, aryl, and aralkyl groups. Preferred are a C₁-C₁₂ linear or branched alkyl group, a C₁-C₁₂ linear or branched alkenyl group, a C₁-C₁₂ linear or branched alkynyl group, a C₃-C₁₂ optionally branched cycloalkyl group, a C₃-C₁₂ optionally branched cycloalkenyl group, a C₆-C₁₂ optionally branched aryl group, and a C₇-C₁₂ optionally branched aralkyl group; and more preferred are a C₁-C₃ linear or branched alkyl group and a C₁-C₃ linear or branched alkenyl group.

In Formula (1), specific examples of the "hydrocarbon group" in R⁴ include methyl, ethyl, propyl, isopropyl, butyl, sec-butyl, isobutyl, tert-butyl, pentyl, neopentyl, hexyl, heptyl, octyl, 2-ethylhexyl, nonyl, decyl, undecyl, dodecyl, vinyl, ethynyl, propenyl, isopropenyl, allyl, propargyl, 1-propynyl, butenyl, crotyl, butadienyl, pentadienyl, cyclopropyl, cyclobutyl, cyclopentyl, cyclohexyl, norbornyl, adamantyl, phenyl, benzyl, tolyl, styryl, 2,4,6-trimethylphenyl, naphthyl, and 2,6-dimethyl-1,5-heptadienyl groups, but are not particularly limited thereto.

In Formula (1), specific examples of the anion represented by R⁴-CO-O⁻ include acetate ion, propionate ion, acrylate ion, propargylate ion, butyrate ion, isobutyrate ion, methacrylate ion, crotonate ion, tetrolate ion, cyclopropanecarboxylate ion, valerate ion, isovalerate ion, pivalate ion, angelate ion, tiglate ion, cyclobutanecarboxylate ion, caproate ion, cyclopentanecarboxylate ion, enanthate ion, sorbate ion, cyclohexanecarboxylate ion, benzoate ion, caprylate ion, 2-ethylhexylcarboxylate ion, toluate ion, 2-norbornenecarboxylate ion, pelargonate ion, cinnamate ion, caprate ion, adamantanecarboxylate ion, geranate ion, undecylate ion, laurate ion, naphthalenecarboxylate ion, and the like; preferably acetate ion, propionate ion, acrylate ion, propargylate ion, butyrate ion, isobutyrate ion, methacrylate ion, crotonate ion, tetrolate ion, valerate ion, isovalerate ion, and pivalate ion; and particularly preferably acetate ion, acrylate ion, or methacrylate ion; however, the present invention is not limited thereto.

In Formula (2), R⁵ represents a C₂-C₅ divalent hydrocarbon group.

The "C₂-C₅ divalent hydrocarbon group" may be linear, branched, or cyclic, or may contain a double bond or a triple bond. Examples include C₂-C₅ alkylene, C₂-C₅ alkenylene, C₂-C₅ alkynylene, C₃-C₅ cycloalkylene, and C₃-C₅ cycloalkenylene groups; preferably C₂ or C₃ alkylene and C₂ or C₃ alkenylene groups; and particularly preferably a C₃ alkylene group.

In Formula (2), specific examples of R⁵ include ethane-1,2-diyl, propane-1,3-diyl, propane-1,2-diyl, cyclopropane-1,2-diyl, butane-1,4-diyl, butane-1,3-diyl, butane-1,2-diyl, cyclobutane-1,3-diyl, cyclobutane-1,2-diyl, 2-methylpropane-1,2-diyl, 2-methylpropane-1,3-diyl, pentane-1,5-diyl, pentane-1,4-diyl, pentane-1,3-diyl, pentane-1,2-diyl, cyclopentane-1,3-diyl, cyclopentane-1,2-diyl, 2-methylbutane-2,4-diyl, 2-methylbutane-1,3-diyl, 2-methylbutane-1,4-diyl, 2,2-dimethylpropane-1,3-diyl, 1,3-butadiene-1,4-diyl, and 2-propene-1,3-diyl groups, but are not particularly limited thereto.

In Formula (2), R⁶ represents a C₁-C₁₀ hydrocarbon group that may contain 1 to 4 heteroatoms.

The "C₁-C₁₀ hydrocarbon group that may contain 1 to 4 heteroatoms" in R⁶ may be linear, branched, or cyclic, or may contain a double bond or a triple bond. Further, this group may not contain a heteroatom or may contain 1 to 4 heteroatoms.

In Formula (2), the number of carbon atoms in the hydrocarbon group that may contain a heteroatom in R⁶ is preferably 1 to 10, more preferably 1 to 8, and even more preferably 1 to 3. When R⁶ is a heteroatom-free hydrocarbon group, the number of carbon atoms is preferably 1 to 10, more preferably 1 to 7, and even more preferably 1 to 3. When R⁶ is a heteroatom-containing hydrocarbon group, the number of carbon atoms is preferably 2 to 10, more preferably 2 to 7, and even more preferably 2 to 4.

In Formula (2), when R⁶ is a hydrocarbon group containing 1 to 4 heteroatoms, examples of the "heteroatom" include nitrogen, oxygen, and sulfur atoms.

In Formula (2), the "C₂-C₁₀ hydrocarbon group containing 1 to 4 heteroatoms" in R⁶ is a heteroatom-free C₂-C₁₀ hydrocarbon group that is interrupted or replaced by a structure, such as - O-, -N<, -NH-, -S-, -C(=O)-O-, -C(=O)-, =N-, -C(=O)-N<, -C(=O)-NH-, -O-C(=O)-O-, -O-C(=O)-N<, -O-C(=O)-NH-, -C(=O)-N[-C(=O)-]-, -C(=O)-NH-C(=O)-, >N-C(=O)-N<, >N-C(=O)-NH-, -HN-C(=O)-NH-, - S(=O)-, -S(=O)₂-, -S(=O)₂-O-, >N-C(=S)-N<, >N-C(=S)-NH-, -HN-C(=S)-NH-, -C(=N-)-, or -C(=NH)-. The number or combination of interruptions or replacements by such structures is not particularly limited as long as the number of heteroatoms in R⁶ is 1 to 4.

Preferred is a hydrocarbon group that is interrupted or replaced by the structure -O- or -S-, and more preferred is a hydrocarbon group that is interrupted or replaced by the structure -O-.

In the present invention, the structure of the hydrocarbon group interrupted or replaced by the structure -O- or -S- may be an ether structure or a thioether structure, and may also be, for example, an epoxy structure or a thioepoxy structure, such as an epoxy group, a thioepoxy group, a glycidyl ether group, or an epoxycyclohexyl group. In this case, R⁶ may be an epoxy group, a thioepoxy group, or the like.

When R⁶ includes two or more structures -O- or/and -S-, the number of carbon atoms between the structures -O- or/and -Sis desirably 2 or more.

In Formula (2), the "heteroatom-free C₂-C₁₀ hydrocarbon group" in R⁶ may be linear, branched, or cyclic, or may contain a double bond or a triple bond. Examples of the heteroatom-free hydrocarbon group include linear or branched alkyl, linear or branched alkenyl, linear or branched alkynyl, optionally branched cycloalkyl, optionally branched cycloalkenyl, optionally branched aryl, and optionally branched aralkyl groups. Preferred are linear or branched alkyl, linear or branched alkenyl, and optionally branched cycloalkyl groups.

Examples of the "C₁-C₁₀ hydrocarbon group that may contain 1 to 4 heteroatoms" include a C₁-C₁₀ linear or branched alkyl group that may contain 1 to 4 heteroatoms, a C₂-C₁₀ linear or branched alkenyl group that may contain 1 to 4 heteroatoms, a C₂-C₁₀ linear or branched alkynyl group that may contain 1 to 4 heteroatoms, a C₃-C₁₀ optionally branched cycloalkyl group that may contain 1 to 4 heteroatoms, a C₃-C₁₀ optionally branched cycloalkenyl group that may contain 1 to 4 heteroatoms, a C₆-C₁₀ optionally branched aryl group containing 1 to 5 heteroatoms, a C₇-C₁₀ optionally branched aralkyl group that may contain 1 to 4 heteroatoms, and the like. Preferred are a C₁-C₁₀ linear or branched alkyl group that may contain 1 to 4 heteroatoms, a C₂-C₁₀ linear or branched alkenyl group that may contain 1 to 4 heteroatoms, and a C₃-C₁₀ optionally branched cycloalkyl group that may contain 1 to 4 heteroatoms.

The "C₁-C₁₀ linear or branched alkyl group that may contain 1 to 4 heteroatoms" is preferably a C₂-C₁₀ linear or branched alkyl group that may contain 1 or 2 heteroatoms. In another embodiment, preferred is a C₂-C₇ linear or branched alkyl group that may contain 1 heteroatom, and more preferred is a C₂-C₄ linear or branched alkyl group that may contain 1 heteroatom. In still another embodiment, preferred is a C₁-C₇ linear or branched alkyl group, and more preferred is a C₁-C₄ linear or branched alkyl group.

The "C₂-C₁₀ linear or branched alkenyl group that may contain 1 to 4 heteroatoms" is preferably a C₂-C₁₀ linear or branched alkenyl group that may contain 1 or 2 heteroatoms. In another embodiment, preferred is a C₂-C₇ linear or branched alkenyl group that may contain 1 heteroatom, and more preferred is a C₂-C₄ linear or branched alkenyl group that may contain 1 heteroatom. In still another embodiment, preferred is a C₂-C₇ linear or branched alkenyl group, and more preferred is a C₂-C₄ linear or branched alkenyl group.

The "C₃-C₁₀ optionally branched cycloalkyl group that may contain 1 to 4 heteroatoms" is preferably a C₃-C₁₀ optionally branched cycloalkyl group that may contain 1 or 2 heteroatoms. In another embodiment, preferred is a C₃-C₇ optionally branched cycloalkyl group that may contain 1 heteroatom, and more preferred is a C₃-C₇ optionally branched cycloalkyl group that may contain 1 heteroatom. In still another embodiment, preferred is a C₃-C₇ optionally branched cycloalkyl group, and more preferred is a C₃ or C₄ optionally branched cycloalkyl group.

In Formula (2), when R⁶ is a heteroatom-free C₁-C₁₀ hydrocarbon group, specific examples of the "C₁-C₁₀ hydrocarbon group" include methyl, ethyl, propyl, isopropyl, butyl, sec-butyl, isobutyl, tert-butyl, pentyl, neopentyl, hexyl, octyl, 2-ethylhexyl, decyl, dodecyl, propenyl, isopropenyl, allyl, propargyl, butenyl, crotyl, butadienyl, geranyl, cyclopropyl, cyclobutyl, cyclopentyl, cyclohexyl, norbornyl, adamantyl, phenyl, benzyl, and 2,4,6-trimethylphenyl groups, but are not particularly limited thereto.

Examples of the "C₂-C₁₀ hydrocarbon group" of the heteroatom-free C₂-C₁₀ hydrocarbon group in R⁶ include ethyl, propyl, isopropyl, butyl, sec-butyl, isobutyl, tert-butyl, pentyl, neopentyl, hexyl, octyl, 2-ethylhexyl, decyl, vinyl, ethynyl, propenyl, isopropenyl, allyl, propargyl, butenyl, crotyl, butadienyl, geranyl, cyclopropyl, cyclobutyl, cyclopentyl, cyclohexyl, norbornyl, adamantyl, phenyl, benzyl, and 2,4,6-trimethylphenyl groups; preferably methyl, ethyl, propyl, isopropyl, butyl, sec-butyl, tert-butyl, pentyl, neopentyl, and hexyl groups; and more preferably methyl, ethyl, propyl, and isopropyl groups. When the heteroatom-free C₂-C₁₀ hydrocarbon group is interrupted or replaced by a structure, such as -O-, -N<, -NH-, -S-, -C(=O)-O-, -C(=O)-, =N-, -C(=O)-N<, - C(=O)-NH-, -O-C(=O)-O-, -O-C(=O) -N<, -O-C(=O)-NH-, -C(=O)-N[-C(=O)-]-, -C(=O)-NH-C(=O)-, >N-C(=O)-N<, >N-C(=O)-NH-, -HN-C(=O)-NH-, -S(=O)-, -S(=O)₂-, -S(=O)₂-O-, >N-C(=S)-N<, >N-C(=S)-NH-, - HN-C(=S)-NH-, -C(=N-)-, or -C(=NH)-, it becomes a "C₂-C₁₀ hydrocarbon group containing 1 to 4 heteroatoms."

In Formula (2), the "heteroatom-free C₂-C₁₀ hydrocarbon group" in R⁶ is preferably a methyl, ethyl, propyl, or isopropyl group, and more preferably a methyl group.

In Formula (2), the "heteroatom-containing C₂-C₁₀ hydrocarbon group" in R⁶ is preferably 2-methoxyethyl, 2-(2-methoxyethoxy)ethyl, furfuryl, thenyl, 2-pyridyl, (oxolan-2-yl)methyl, 2-morpholinoethyl, 2-(dimethylamino)ethyl, or 1,2-epoxypropyl; and more preferably 2-methoxyethyl; however, the present invention is not limited thereto.

In Formula (2), R⁶ is preferably a methyl, ethyl, propyl, or isopropyl group; and more preferably a methyl group.

In Formula (2), the total number of carbon atoms in R⁵ and R⁶ is 3 to 12.

In Formula (2), X representing an oxygen or sulfur atom means that R⁵ and R⁶ are bonded via an ether group (-O-) or a thioether group (-S-).

Specific examples of the imidazolium cation of the onium salt represented by Formula (1) include 1,3-bis(2-methoxyethyl)imidazolium, 1,3-bis(2-ethoxyethyl)imidazolium, 1,3-bis(2-propoxyethyl)imidazolium, 1,3-bis(2-isopropoxyethyl)imidazolium, 1,3-bis(2-phenoxyethyl)imidazolium, 1,3-bis(2-benzyloxyethyl)imidazolium, 1,3-bis[2-(p-methoxybenzyloxy)ethyl]imidazolium, 1,3-bis[2-(2-methoxyethoxymethoxy)ethyl]imidazolium, 1,3-bis[2-(2-benzyloxyethoxy)ethyl]imidazolium, 1,3-bis(3-methoxypropyl)imidazolium, 1,3-bis(3-ethoxypropyl)imidazolium, 1,3-bis(3-propoxypropyl)imidazolium, 1,3-bis(3-isopropoxypropyl)imidazolium, 1,3-bis(3-allyloxypropyl)imidazolium, 1,3-bis(3-butoxypropyl)imidazolium, 1,3-bis[3-(1-methylpropoxy)propyl]imidazolium, 1,3-bis[3-(2-methylpropoxy)propyl]imidazolium, 1,3-bis[3-(1,1-dimethylethoxy)propyl]imidazolium, 1,3-bis[(oxolan-2-yl)methyl]imidazolium, 1,3-bis(3-pentyloxypropyl)imidazolium, 1,3-bis(3-neopentyloxypropyl)imidazolium, 1,3-bis(3-hexyloxypropyl)imidazolium, 1,3-bis(3-cyclohexyloxypropyl)imidazolium, 1,3-bis(3-heptyloxypropyl)imidazolium, 1,3-bis(3-octyloxypropyl)imidazolium, 1,3-bis(3-nonyloxypropyl)imidazolium, 1,3-bis{3-[(2-ethylhexan-1-yl)oxy]propyl}imidazolium, 1,3-bis[3-(2,4,6-trimethylphenoxy)propyl]imidazolium, 1,3-bis[3-(2-methoxyethoxy)propyl]imidazolium, 1,3-bis[2-(dimethylamino)ethyl]imidazolium, 1,3-bis[3-(dimethylamino)propyl]imidazolium, 1,3-bis(2-methylthioethyl)imidazolium, 1,3-bis{2-[(furan-2-yl)methylthio]ethyl]imidazolium, 1,3-bis[2-(furan-2-yl)methyl]imidazolium, 1,3-bis[1-(methoxymethyl)propyl]imidazolium, 1,3-bis(4-methoxybutyl)imidazolium, 1,3-bis(4-ethoxybutyl)imidazolium, 1,3-bis(4-propoxybutyl)imidazolium, 1,3-bis(4-isopropoxybutyl)imidazolium, 1,3-bis(4-allyloxybutyl)imidazolium, 1,3-bis(4-butoxybutyl)imidazolium, 1,3-bis[4-(1-methylpropoxy)butyl]imidazolium, 1,3-bis[4-(2-methylpropoxy)butyl]imidazolium, 1,3-bis[4-(1,1-dimethylethoxy)butyl]imidazolium, 1,3-bis(4-pentyloxybutyl)imidazolium, 1,3-bis(4-neopentyloxybutyl)imidazolium, 1,3-bis(4-hexyloxybutyl)imidazolium, 1,3-bis(4-cyclohexyloxybutyl)imidazolium, 1,3-bis(4-heptyloxybutyl)imidazolium, 1,3-bis(4-octyloxybutyl)imidazolium, 1,3-bis{4-[(2-ethylhexan-1-yl)oxy]butyl}imidazolium, 1,3-bis(5-methoxypentyl)imidazolium, 1,3-bis(5-ethoxypentyl)imidazolium, 1,3-bis(5-propoxypentyl)imidazolium, 1,3-bis(5-isopropoxypentyl)imidazolium, 1,3-bis(5-allyloxypentyl)imidazolium, 1,3-bis(5-butoxypentyl)imidazolium, 1,3-bis[5-(1-methylpropoxy)pentyl]imidazolium, 1,3-bis[5-(2-methylpropoxy)pentyl]imidazolium, 1,3-bis[5-(1,1-dimethylethoxy)pentyl]imidazolium, 1,3-bis(5-pentyloxypentyl)imidazolium, 1,3-bis(5-neopentyloxypentyl)imidazolium, 1,3-bis(5-hexyloxypentyl)imidazolium, 1,3-bis(5-cyclohexyloxypentyl)imidazolium, 1,3-bis(5-heptyloxypentyl)imidazolium, 1,3-bis(6-methoxyhexyl)imidazolium, 1,3-bis(6-ethoxyhexyl)imidazolium, 1,3-bis(6-propoxyhexyl)imidazolium, 1,3-bis(6-isopropoxyhexyl)imidazolium, 1,3-bis(6-allyloxyhexyl)imidazolium, 1,3-bis(6-butoxyhexyl)imidazolium, 1,3-bis[6-(1-methylpropoxy)hexyl]imidazolium, 1,3-bis[6-(2-methylpropoxy)hexyl]imidazolium, 1,3-bis[6-(1,1-dimethylethoxy)hexyl]imidazolium, 1,3-bis(6-pentyloxyhexyl)imidazolium, 1,3-bis(6-neopentyloxyhexyl)imidazolium, 1,3-bis(6-hexyloxyhexyl)imidazolium, 1,3-bis(6-cyclohexyloxyhexyl)imidazolium, 1,3-bis[(2-methoxycyclohexan-1-yl)methyl]imidazolium, 1,3-bis[(2-ethoxycyclohexan-1-yl)methyl]imidazolium, 1,3-bis[(2-propoxycyclohexan-1-yl)methyl]imidazolium, 1,3-bis[(2-isopropoxycyclohexan-1-yl)methyl]imidazolium, 1,3-bis[(2-butoxycyclohexan-1-yl)methyl]imidazolium, 1,3-bis{[2-(1-methylpropoxy)cyclohexan-1-yl]methyl}imidazolium, 1,3-bis{[2-(2-methylpropoxy)cyclohexan-1-yl]methyl}imidazolium, 1,3-bis{[2-(1,1-dimethylethoxy)cyclohexan-1-yl]methyl}imidazolium, 1,3-bis[(2-pentyloxycyclohexan-1-yl)methyl]imidazolium, 1,3-bis[(2-neopentyloxycyclohexan-1-yl)methyl]imidazolium, 1,3-bis(3-methylthiopropyl)imidazolium, 1,3-bis[2-(methylsulfonyl)ethyl]imidazolium, 1,3-bis[(thiophen-2-yl)methyl]imidazolium, 1,3-bis(p-methoxyphenyl)imidazolium, 1,3-bis(p-ethoxyphenyl)imidazolium, 1,3-bis(3,4-methylenedioxyphenyl)imidazolium, 1,3-bis(3,4-methylenedioxybenzyl)imidazolium, 1,3-bis{(7-oxabicyclo[2,2,1]heptan-2-yl)methyl}imidazolium, 1-methoxypropyl-3-methylthiopropylimidazolium, 1,3-bis(3-methoxypropyl)-2-methylimidazolium, 1,3-bis(2-methoxyethyl)-2-methylimidazolium, and the like; preferably 1,3-bis(3-methoxypropyl)imidazolium, 1,3-bis(3-ethoxypropyl)imidazolium, 1,3-bis(3-propoxypropyl)imidazolium, 1,3-bis(3-isopropoxypropyl)imidazolium, 1,3-bis(3-allyloxypropyl)imidazolium, 1,3-bis(3-butoxypropyl)imidazolium, 1,3-bis[3-(1-methylpropoxy)propyl]imidazolium, 1,3-bis[3-(2-methylpropoxy)propyl]imidazolium, 1,3-bis[3-(1,1-dimethylethoxy)propyl]imidazolium, 1,3-bis[(oxolan-2-yl)methyl]imidazolium, 1,3-bis[3-(2-methoxyethoxy)propyl]imidazolium, 1,3-bis(3-methylthiopropyl)imidazolium, 1-methoxypropyl-3-methylthiopropylimidazolium, 1,3-bis(3-methoxypropyl)-2-methylimidazolium, and 1,3-bis(2-methoxyethyl)-2-methylimidazolium; and more preferably 1,3-bis(3-methoxypropyl)imidazolium, 1,3-bis(3-ethoxypropyl)imidazolium, **1-**methoxypropyl-3-methylthiopropylimidazolium, 1,3-bis[3-(2-methoxyethoxy)propyl]imidazolium, 1,3-bis(2-methoxyethyl)-2-methylimidazolium, and 1,3-bis(3-methoxypropyl)-2-methylimidazolium; however, the present invention is not limited thereto.

The onium salt represented by Formula (1) is preferably an onium salt made of a combination of one cation selected from the group consisting of 1,3-bis(2-methoxyethyl)imidazolium, 1,3-bis(3-methoxypropyl)imidazolium, 1,3-bis(3-ethoxypropyl)imidazolium, 1,3-bis(3-methylthiopropyl)imidazolium, 1,3-bis[3-(2-methoxyethoxy)propyl]imidazolium, 1-methoxypropyl-3-methylthiopropylimidazolium, 1,3-bis(2-methoxyethyl)-2-methylimidazolium, and 1,3-bis(3-methoxypropyl)-2-methylimidazolium, and one anion selected from the group consisting of acetate ion, acrylate ion, and methacrylate ion.

More specific examples of the onium salt represented by Formula (1) include 1,3-bis(2-methoxyethyl)imidazolium acetate, 1,3-bis(3-methoxypropyl)imidazolium acetate, 1,3-bis(3-methoxypropyl)imidazolium acrylate, 1,3-bis(3-methoxypropyl)imidazolium methacrylate, 1,3-bis(3-ethoxypropyl)imidazolium acetate, 1-methoxypropyl-3-methylthiopropylimidazolium acetate, 1,3-bis(3-methylthiopropyl)imidazolium acetate, 1,3-bis(2-methoxyethyl)-2-methylimidazolium acetate, 1,3-bis(2-methoxyethyl)-2-methylimidazolium acrylate, 1,3-bis(2-methoxyethyl)-2-methylimidazolium methacrylate, 1,3-bis(3-methoxypropyl)-2-methylimidazolium acetate, 1,3-bis(3-methoxypropyl)-2-methylimidazolium acrylate, 1,3-bis(3-methoxypropyl)-2-methylimidazolium methacrylate, 1,3-bis[3-(2-methoxyethoxy)propyl]imidazolium acetate, 1,3-bis[3-(2-methoxyethoxy)propyl]imidazolium acrylate, 1,3-bis[3-(2-methoxyethoxy)propyl]imidazolium methacrylate, and the like; and preferably 1,3-bis(3-methoxypropyl)imidazolium acetate, 1,3-bis(3-methoxypropyl)imidazolium acrylate, and 1,3-bis(3-methoxypropyl)imidazolium methacrylate; however, the present invention is not limited thereto.

The onium salt (1) of the present invention would have easily been produced by a person skilled in the art based on the method described in the Examples. The onium salt (1) can be obtained, for example, by the production method shown in the following Reaction Formula 1 (Production Method 1).
Reaction Formula 1: 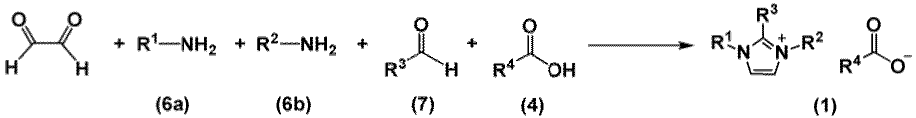 wherein in Reaction Formula 1, R¹, R², R³, and R⁴ are each as defined above.

Production Method 1 is explained. Glyoxal, an amine compound represented by R¹-NH₂ (Formula (6a)) and/or R²-NH₂ (Formula (6b)), an aldehyde compound represented by R³-CHO (Formula (7)), and a carboxylic acid represented by R⁴-COOH (Formula (4)) are reacted.

Specific examples of the amine compound represented by R¹-NH₂ (Formula (6a)) or R²-NH₂ (Formula (6b)) include 2-methoxyethylamine, 2-ethoxyethylamine, 2-propoxyethylamine, 2-isopropoxyethylamine, 2-allyloxyethylamine, 2-butoxyethylamine, 2-(1-methylpropoxy)ethylamine, 2-(2-methylpropoxy)ethylamine, 2-(1,1-dimethylethoxy)ethylamine, 2-pentyloxyethylamine, 2-neopentyloxyethylamine, 2-hexyloxyethylamine, 2-phenoxyethylamine, 2-heptyloxyethylamine, 2-octyloxyethylamine, 2-nonyloxyethylamine, 2-decyloxyethylamine, 2-cyclopentyloxyethylamine, 2-cyclohexyloxyethylamine, 2-(2-ethylhexan-1-yl)oxyethylamine, 2-(2,4,6-trimethylphenoxy)ethylamine, 2-(benzyloxy)ethylamine, 2-(p-methoxybenzyloxy)ethylamine, 2-(2-methoxyethoxymethoxy)ethylamine, 2-(2-benzyloxyethoxy)ethylamine, 2-(dimethylamino)ethylamine, 3-methoxypropylamine, 3-ethoxypropylamine, 3-propoxypropylamine, 3-allyloxypropylamine, 3-isopropoxypropylamine, 3-(1-methylpropoxy)propylamine, 3-(2-methylpropoxy)propylamine, 3-(1,1-dimethylethoxy)propylamine, 3-butoxypropylamine, 3-pentyloxypropylamine, 3-neopentyloxypropylamine, 3-hexyloxypropylamine, 3-cyclohexyloxypropylamine, 3-heptyloxypropylamine, 3-octyloxypropylamine, 3-nonyloxypropylamine, 3-(2-ethylhexan-1-yl)oxypropylamine, 3-(2,4,6-trimethylphenoxy)propylamine, 3-(2-methoxyethoxy)propylamine, 3-(dimethylamino)propylamine, 4-methoxybutylamine, 4-ethoxybutylamine, 4-propoxybutylamine, 4-isopropoxybutylamine, 4-allyloxybutylamine, 4-butoxybutylamine, 4-(1-methylpropoxy)butylamine, 4-(2-methylpropoxy)butylamine, 4-(1,1-dimethylethoxy)butylamine, 4-pentyloxybutylamine, 4-neopentyloxybutylamine, 4-hexyloxybutylamine, 4-cyclohexyloxybutylamine, 4-heptyloxybutylamine, 4-octyloxybutylamine, 4-(2-ethylhexan-1-yl)oxybutylamine, 5-methoxypentylamine, 5-ethoxypentylamine, 5-propoxypentylamine, 5-isopropoxypentylamine, 5-allyloxypentylamine, 5-butoxypentylamine, 5-(1-methylpropoxy)pentylamine, 5-(2-methylpropoxy) pentylamine, 5-(1,1-dimethylethoxy)pentylamine, 5-pentyloxypentylamine, 5-neopentyloxypentylamine, 5-hexyloxypentylamine, 5-cyclohexyloxypentylamine, 5-heptyloxypentylamine, 6-methoxyhexylamine, 6-ethoxyhexylamine, 6-propoxyhexylamine, 6-isopropoxyhexylamine, 6-allyloxyhexylamine, 6-butoxyhexylamine, 6-(1-methylpropoxy)hexylamine, 6-(2-methylpropoxy)hexylamine, 6-(1,1-dimethylethoxy)hexylamine, 6-pentyloxyhexylamine, 6-neopentyloxyhexylamine, 6-hexyloxyhexylamine, 6-cyclohexyloxyhexylamine, 2-methoxycyclohexyl-1-ylmethylamine, (2-ethoxycyclohexan-1-yl)methylamine, (2-propoxycyclohexan-1-yl)methylamine, (2-isopropoxycyclohexan-1-yl)methylamine, (2-butoxycyclohexyl-1-yl)methylamine, [2-(1-methylpropoxy)cyclohexan-1-yl]methylamine, [2-(2-methylpropoxy)cyclohexan-1-yl]methylamine, [2-(1,1-dimethylethoxy)cyclohexan-1-yl]methylamine, (2-pentyloxycyclohexan-1-yl)methylamine, (2-neopentyloxycyclohexan-1-yl)methylamine, 2-methylthioethylamine, 3-methylthiopropylamine, 1-(methoxymethyl)propylamine, (oxolan-2-yl)methylamine, (furan-2-yl)methylamine, 2-[(furan-2-yl)methylthio]ethylamine, 2-(methylsulfonyl)ethylamine, (thiophen-2-yl)methylamine, p-methoxyphenylamine, p-ethoxyphenylamine, 3,4-methylenedioxyaniline, 3,4-methylenedioxybenzylamine, (7-oxabicyclo[2,2,1]heptan-2-yl)methylamine, and the like; preferably 2-methoxyethylamine, 3-methoxypropylamine, 3-ethoxypropylamine, 3-isopropoxypropylamine, 3-butoxypropylamine, 3-(2-methoxyethoxy)propylamine, 3-methylthiopropylamine, and 3-(dimethylamino)propylamine; and more preferably 2-methoxyethylamine, 3-methoxypropylamine, and 3-(2-methoxyethoxy)propylamine; however, the present invention is not limited thereto. The amine compounds represented by R¹-NH₂ or R²-NH₂ may be the same or different.

Specific examples of the aldehyde compound represented by R³-CHO (Formula (7)) include formaldehyde, acetaldehyde, propionaldehyde, butanal, pentanal, hexanal, heptanal, octanal, nonanal, decanal, acrolein, benzaldehyde, cinnamaldehyde, perillaldehyde, vanillin, and the like; and preferably formaldehyde and acetaldehyde; however, the present invention is not limited thereto. As the formaldehyde, a cyclic oligomer or polymer capable of producing formaldehydes, such as paraformaldehyde, trioxane, and tetraoxane, in the reaction system may be used.

As the aldehyde compound represented by R³-CHO (Formula (7)), an aqueous solution or an alcohol solution, such as methanol, butanol, or 2-ethylhexanol, may be used as is.

Examples of the carboxylic acid represented by R⁴-COOH (Formula (4)) include acetic acid, propionic acid, acrylic acid, propargylic acid, butyric acid, isobutyric acid, methacrylic acid, crotonic acid, tetronic acid, cyclopropanecarboxylic acid, valeric acid, isovaleric acid, pivalic acid, angelic acid, tiglic acid, cyclobutanecarboxylic acid, caproic acid, cyclopentanecarboxylic acid, enanthic acid, sorbic acid, cyclohexanecarboxylic acid, benzoic acid, caprylic acid, 2-ethylhexylcarboxylic acid, toluic acid, 2-norbornanecarboxylic acid, pelargonic acid, cubanecarboxylic acid, cinnamic acid, capric acid, 3-noradamantanecarboxylic acid, geranic acid, undecyl acid, 1-naphthalenecarboxylic acid, 2-naphthalenecarboxylic acid, 1-adamantanecarboxylic acid, and the like; preferably acetic acid, propionic acid, acrylic acid, propargylic acid, butyric acid, isobutyric acid, methacrylic acid, crotonic acid, tetronic acid, valeric acid, isovaleric acid, and pivalic acid; and particularly preferably acetic acid, acrylic acid, or methacrylic acid; however, the present invention is not limited thereto.

As glyoxal, an aqueous solution or an alcohol solution, such as methanol, butanol, or 2-ethylhexanol, may be used as is.

As for the amounts of the amine compounds represented by R¹-NH₂ (Formula (6a)) and R²-NH₂ (Formula (6b)) used, the total amount of R¹-NH₂ and R²-NH₂ is generally 0.1 to 10 mol, and preferably 0.5 to 3 mol, per mol of glyoxal. When R¹ and R² are different, the ratio (molar ratio) of the two amine compounds (R¹-NH₂ and R²-NH₂) is not particularly limited, and is such that the ratio of R¹-NH₂ to R²-NH₂ is 0:100 to 100:0. When the ratio of R¹-NH₂ to R²-NH₂ is 0:100 or 100:0, R¹ = R². When the ratio of R¹-NH₂ to R²-NH₂ is not 0:100 or 100:0, the onium salt (1) obtained by Production Method 1 is a mixture of compounds represented by the following formulas. The compounds represented by the following Formulas (1') and (1'') are both included in the onium salt represented by Formula (1), and the mixture may be used as an onium salt composition, described later. If necessary, the mixture can be purified to form a desired single compound, which can then be used as the onium salt (1).

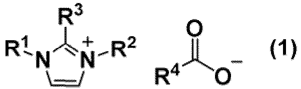

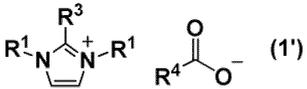

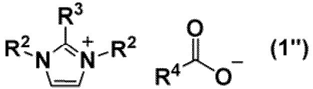

As the aldehyde compound represented by R³-CHO (Formula (7)), an aqueous solution or an alcohol solution, such as methanol, butanol, or 2-ethylhexanol, may be used as is. The amount of the aldehyde compound used is generally 0.1 to 10 mol, and preferably 0.5 to 5 mol, per mol of glyoxal.

The amount of the carboxylic acid represented by R⁴-COOH (Formula (4)) used is generally 0.1 to 10 mol, and preferably 0.5 to 2 mol, per mol of glyoxal.

In Production Method 1, a solvent may not be used. When a solvent is used, the solvent used is not particularly limited as long as it does not affect the reaction. Specific examples of solvents include aromatic hydrocarbon solvents, such as toluene, benzene, and xylene; hydrocarbon solvents, such as methylcyclohexane, cyclohexane, hexane, heptane, and octane; halogenated hydrocarbon solvents, such as dichloromethane and chloroform; ether solvents, such as diethyl ether, tetrahydrofuran, and 1,4-dioxane; alcohol solvents, such as methanol, ethanol, and 2-propanol; N,N-dimethylformamide, acetonitrile, water, and the like; and preferably alcohol solvents and water.

Further, the onium salt (1) produced in advance can be used as a reaction solvent in Production Method 1. If necessary, two or more solvents can be used as a mixture. The amount of solvent used is generally 50 parts by mass or less, and preferably 0.1 to 10 parts by mass, per part by mass of glyoxal.

In Production Method 1, the reaction may be performed, if necessary, in an inert gas atmosphere, such as nitrogen, argon, or helium, which do not affect the reaction.

When Production Method 1 is performed, the reaction pressure is generally atmospheric pressure; however, depending on the raw materials and solvents used, the reaction may be carried out by pressurizing the reaction system from atmospheric pressure to 1 MPa.

The reaction temperature varies depending on the raw materials, solvents, etc. used, and is generally -10°C or more, and preferably 0°C to 100°C.

After completion of the reaction in Production Method 1, the onium salt (1) may be purified by a known purification method or may not be purified. Examples of the purification method include concentration, liquid separation, recrystallization, dialysis, adsorption, filtration, and the like, but are not particularly limited thereto.

When the anion represented by R⁴-COO⁻ in the onium salt (1) obtained in Production Method 1 is not a desired anion, the ion exchange reaction may be further performed, if necessary, to obtain the desired anion. In this case, for example, Production Method 3 described later can be preferably used. When Production Method 3 is used, ion exchange is performed using the onium salt (1) obtained in Production Method 1 as an onium salt represented by Formula (2) in Production Method 3, whereby an onium salt (1) having the desired anion can be produced.

Further, the onium salt (1) can also be obtained by exchanging (ion-exchanging) Y⁻ in an onium salt represented by the following Formula (2) (hereinafter referred to as the onium salt (2)) with a desired R⁴-COO⁻ anion.
Formula (2): 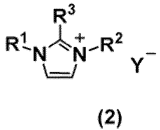 wherein R¹, R², and R³ are as defined above, and Y⁻ represents any anion.

Examples of Y⁻ in Formula (2) include halogen anions, such as chloride ion, bromide ion, and iodide ion; hydroxide anion, carboxylate anion, sulfonate anion, thiocyanate anion, nitrate anion, perchlorate anion, and the like, but are not particularly limited thereto. When Y⁻ is a carboxylate anion, it is not the same as the R⁴-COO⁻ anion in the onium salt (1) represented by Formula (1).

Examples of the method of ion exchange include a direct exchange method as shown in Reaction Formula 2 below (Production Method 2). In addition, when Y⁻ in Formula (2) is not a hydroxide anion, a method of ion exchange via a hydroxide salt (3) as shown in Reaction Formula 3 below (Production Method 3) can also be used.
Reaction Formula 2: 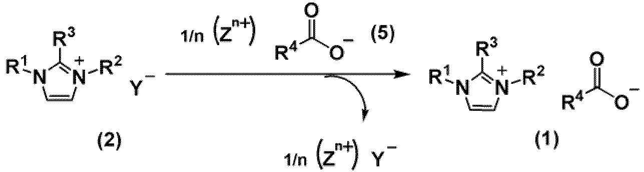 wherein in Reaction Formula 2, R¹, R², R³, R⁴, and Y⁻ are each as defined above, n represents any natural number (preferably a natural number of 1 to 4, more preferably 1, 2, or 3), and Zⁿ⁺ represents any cation.
Reaction Formula 3: 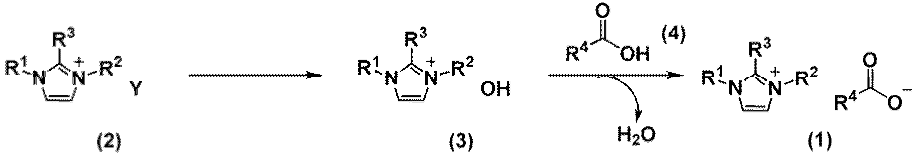 wherein in Reaction Formula 3, R¹, R², R³, R⁴, and Y⁻ are each as defined above, provided that in Reaction Formula 3, Y⁻ in Formula (2) represents any anion other than hydroxide anions.

In Reaction Formula 2, examples of the cation represented by Zⁿ⁺ include alkali metal ions, such as lithium cation, sodium cation, and potassium cation; alkaline earth metal ions, such as calcium cation; ammonium cations, and the like, but are not particularly limited thereto.

Production Method 3 is explained.

Production Method 3 includes a reaction to perform ion exchange of an onium salt (2) represented by Formula (2) using an ion exchange resin, silver(I) oxide, an alkali metal hydroxide, or the like to produce an onium hydroxide salt represented by Formula (3) (hereinafter referred to as the hydroxide salt (3)) (hereinafter referred to as the first reaction), and a reaction to perform ion exchange by reacting the resulting hydroxide salt (3) with a carboxylic acid represented by Formula (4) to obtain an onium salt (1) (hereinafter referred to as the second reaction).

A case in which an ion exchange resin is used in the first reaction (Reaction 1-1) is explained. The ion exchange resin used in Reaction 1-1 can be, for example, a strongly basic ion exchange resin (OH-type) commercially available for water treatment or catalyst use. The amount of ion exchange resin used is generally 20 molar equivalents or less, and preferably 1 to 10 molar equivalents, per mol of the onium salt (2).

Examples of the method for bringing the onium salt (2) into contact with an ion exchange resin include a method in which the onium salt (2) is passed through a column packed with the ion exchange resin (Reaction 1-1-1), and a method in which the onium salt (2) and the ion exchange resin are mixed (Reaction 1-1-2).

In Reaction 1-1, the onium salt (2) is generally diluted with a solvent and then brought into contact with the ion exchange resin. Examples of solvents include acetone; alcohols, such as methanol, ethanol, and 2-propanol; acetonitrile, ethyl acetate, water, and the like.

The amount of solvent used is not particularly limited. In the case of Reaction 1-1-1, the amount of solvent used is generally 1 to 100 parts by mass, preferably 2 to 20 parts by mass, and more preferably 3 to 10 parts by mass, per part by mass of the onium salt (2). In the case of Reaction 1-1-2, the amount of solvent used is generally 10 parts by mass or less, and preferably 1 to 10 parts by mass, per part by mass of the onium salt (2).

A case in which silver(I) oxide is used in the first reaction (Reaction 1-2) is explained. The amount of silver(I) oxide used in Reaction 1-2 is generally 10 molar equivalents or less, and preferably 0.5 to 2 molar equivalents, per mol of the onium salt (2).

A case in which an alkali metal hydroxide is used in the first reaction (Reaction 1-3) is explained. Examples of the alkali metal hydroxide used in Reaction 1-3 include sodium hydroxide, potassium hydroxide, and the like. The amount of the alkali metal hydroxide used is generally 3 molar equivalents or less per mol of the onium salt (2).

Reactions 1-2 and 1-3 are generally performed in a solvent. Examples of solvents include acetone; alcohols, such as methanol, ethanol, and 2-propanol; acetonitrile, ethyl acetate, water, and the like. The amount of solvent used is not particularly limited, but is generally 10 parts by mass or less, and preferably 1 to 10 parts by mass, per part by mass of the onium salt (2).

The reaction temperature of the first reaction is generally 10°C or more, preferably 10 to 60°C, and particularly preferably 10 to 30°C.

The reaction mixture obtained in the first reaction may be directly used in the second reaction, or the reaction liquid may be filtered to remove insoluble substances, and then used in the second reaction.

Examples of the hydroxide salt (3) obtained in the first reaction include compounds in which the R⁴-COO⁻ anion of the onium salt represented by Formula (1) is replaced by an OH⁻ anion (compounds represented by Formula (3)).

Specific examples include 1,3-bis(2-methoxyethyl)imidazolium hydroxide, 1,3-bis(3-methoxypropyl)imidazolium hydroxide, 1,3-bis(3-ethoxypropyl)imidazolium hydroxide, 1,3-bis(3-propoxypropyl)imidazolium hydroxide, 1,3-bis(3-isopropoxypropyl)imidazolium hydroxide, 1,3-bis(3-allyloxypropyl)imidazolium hydroxide, 1,3-bis(3-butoxypropyl)imidazolium hydroxide, 1,3-bis[3-(1-methylpropoxy)propyl]imidazolium hydroxide, 1,3-bis[3-(2-methylpropoxy)propyl]imidazolium hydroxide, 1,3-bis[3-(1,1-dimethylethoxy)propyl]imidazolium hydroxide, 1,3-bis[(oxolan-2-yl)methyl]imidazolium hydroxide, 1,3-bis[3-(2-methoxyethoxy)propyl]imidazolium hydroxide, 1,3-bis(3-methylthiopropyl)imidazolium hydroxide, 1-methoxypropyl-3-methylthiopropylimidazolium hydroxide, 1,3-bis(3-methoxypropyl)-2-methylimidazolium hydroxide, 1,3-bis(2-methoxyethyl)-2-methylimidazolium hydroxide, and the like; and preferably 1,3-bis(2-methoxyethyl)imidazolium hydroxide, 1,3-bis(3-methoxypropyl)imidazolium hydroxide, 1,3-bis(3-ethoxypropyl)imidazolium hydroxide, 1-methoxypropyl-3-methylthiopropylimidazolium hydroxide, 1,3-bis[3-(2-methoxyethoxy)propyl]imidazolium hydroxide, 1,3-bis(2-methoxyethyl)-2-methylimidazolium hydroxide, and 1,3-bis(3-methoxypropyl)-2-methylimidazolium hydroxide; however, the present invention is not limited thereto.

Next, the second reaction is explained. In the second reaction, the carboxylic acid represented by Formula (4) can be the same as the carboxylic acid in Production Method 1. The amount of the carboxylic acid represented by Formula (4) used in the second reaction is generally 0.8 molar equivalents or more, preferably 0.8 to 1.2 molar equivalents, and particularly preferably 0.9 to 1.1 molar equivalents, per mol of the hydroxide salt (3) represented by Formula (3).

The second reaction is generally performed in a solvent. Examples of solvents include ketones, such as acetone and methyl ethyl ketone; alcohols, such as methanol, ethanol, and 2-propanol; acetonitrile, ethyl acetate, tetrahydrofuran, dimethylformamide, water, and the like. The amount of solvent used is not particularly limited, but is generally 10 parts by mass or less, and preferably 1 to 10 parts by mass, per part by mass of the hydroxide salt (3).

The reaction temperature of the second reaction is generally 10°C or more, preferably 10 to 60°C, and particularly preferably 10 to 30°C.

After completion of the second reaction, for example, the obtained reaction mixture can be concentrated to thereby obtain an onium salt (1). The resulting onium salt (1) may be purified by a known purification method, or may not be purified. Examples of the purification method include concentration, liquid separation, recrystallization, dialysis, adsorption, filtration, and the like, but are not particularly limited thereto.

The onium salt (2) can be obtained, for example, by the production method shown in the following Reaction Formula 4 (Production Method 4).
Reaction Formula 4: 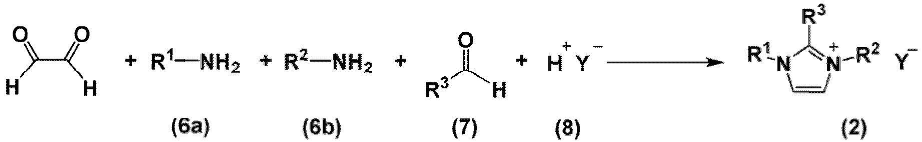 wherein in Reaction Formula 4, R¹, R², R³, and Y⁻ are each as defined above.

In Production Method 4, glyoxal, an amine compound represented by R¹-NH₂ (Formula (6a)) and/or R²-NH₂ (Formula (6b)), an aldehyde compound represented by R³-CHO (Formula (7)), and an acid represented by H⁺Y⁻ (Formula (8)) are reacted.

Production Method 4 can be performed in the same manner as Production Method 1, except that the acid represented by H⁺Y⁻(Formula (8)) is used in place of the carboxylic acid represented by R⁴-COOH (Formula (4)) in Production Method 1. That is, the amine compound represented by R¹-NH₂ (Formula (6a)) or R²-NH₂ (Formula (6b)), the aldehyde compound represented by R³-CHO (Formula (7)), the reaction pressure, the reaction temperature, the post-treatment after completion of the reaction, the purification method, and the like are the same as those in Production Method 1. Further, as in Production Method 1, the reaction may be performed, if necessary, in an inert gas atmosphere, such as nitrogen, argon, or helium, which do not affect the reaction.

Examples of the acid represented by H⁺Y⁻ (Formula (8)) include hydrogen halides, such as hydrogen chloride, hydrogen bromide, and hydrogen iodide; carboxylic acid, sulfonic acid, thiocyanic acid, nitric acid, perchloric acid, and the like. Such an acid, even in the form of an aqueous solution or an organic solvent solution, can be used in Production Method 4.

When Y⁻ in the onium salt (2) is a halogen anion, it can also be produced by the production method shown in the following Reaction Formula 5 (production method 5).
Reaction Formula 5: 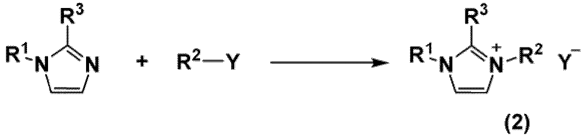 wherein in Reaction Formula 5, R¹ to R³ are as defined above, Y is a halogen atom, and Y⁻ is a halogen anion.

In Reaction Formula 5, examples of the halogen atom represented by Y include chlorine, bromine, and iodine atoms, and examples of the halogen atom represented by Y⁻ include chloride, bromide, and iodide anions, but are not particularly limited thereto.

The onium salt composition in one embodiment of the present invention contains two or more onium salts (1).

Hereinafter, the cation of the onium salt represented by Formula (1) is referred to as "imidazolium cation (C⁺)," and the anion of the onium salt represented by Formula (1) is referred to as "carboxylate anion (A⁻)." The onium salt composition may contain two or more imidazolium cations (C⁺) and two or more carboxylate anions (A⁻), may contain two or more imidazolium cations (C⁺) and one carboxylate anion (A⁻), or may contain one imidazolium cation (C⁺) and two or more carboxylate anions (A⁻).

The onium salt composition can be produced, for example, by mixing two or more onium salts (1). Examples of the method for mixing two or more onium salts (1) include direct mixing at any temperature, as well as use of a co-solvent, salt exchange by dialysis or using an ion exchange resin, and the like, but are not particularly limited thereto.

Further, for example, two or more onium salts (1) can be produced by the following method (i) or (ii) to obtain an onium salt composition.
(i) In Production Method 1, the reaction is performed with any one or a combination of the following (a), (b), and (c):
   (a) using, as the amine compound, amine compounds represented by R¹-NH₂ and R²-NH₂ (different from R¹ and R²)
   (b) using, as the aldehyde compound, two or more aldehyde compounds represented by R³-CHO
   (c) using, as the carboxylic acid, two or more carboxylic acids represented by R⁴-COOH (4)
(ii) In Production Method 1, an onium salt (1) produced in advance is used as a reaction solvent, and after completion of the reaction, the onium salt (1) used as the reaction solvent is not removed. In this case, the onium salt (1) produced in advance is not the same as an onium salt (1) obtained through Production Method 1 using the onium salt (1) produced in advance as the solvent.

The polysaccharides in the present invention are substances composed of a large number of monosaccharide molecules polymerized by glycosidic bonds. Examples of constituent monosaccharide molecules include glucose, fructose, glucosamine, N-acetylglucosamine, and the like. Glucose-derived polysaccharides include glycogen, starch, cellulose, dextrin, and curdlan, fructose-derived polysaccharides include fructan, and glucosamine-derived polysaccharides include chitin and chitosan. In particular, poorly soluble cellulose, curdlan, chitin, chitosan, and the like are suitable for the present invention.

Cellulose refers to a polymer of glucose polymerized by β-1,4-glucosidic bonds and derivatives of the polymer. The degree of polymerization of glucose in cellulose is not particularly limited, but is preferably 200 or more. Examples of derivatives include carboxymethylation, aldehydation, and esterification derivatives. The cellulose may also contain cello-oligosaccharides and cellobiose, which are partial decomposition products of cellulose. Further, the cellulose may be a composite with β-glucoside (glycoside), lignocellulose, which is lignin and/or hemicellulose, or a composite with pectin or the like. The onium salt of the present invention can dissolve the cellulose at 10°C to 50°C even if it is lignocellulose or a composite with pectin or the like. The cellulose may be crystalline cellulose or non-crystalline cellulose, but is preferably crystalline cellulose. Further, the cellulose may be naturally derived or artificially synthesized. The origin of the cellulose is also not limited. The cellulose may be of plant, fungal, or bacterial origin.

The cellulose also includes cellulose-containing materials. Specific examples of cellulose-containing materials include pulp, natural fibers such as cotton and hemp, recycled fibers such as rayon, cupra, and acetate, rice straw and other types of straw, agricultural wastes such as bagasse and wood chips, and biomass including waste paper, construction wastes, and other various wastes. When the pulp, natural fibers such as cotton and hemp, recycled fibers such as rayon, cupra, and acetate, rice straw and other types of straw, agricultural wastes such as bagasse and wood chips, and biomass including waste paper, construction wastes, and other various wastes mentioned above are dissolved in the onium salt of the present invention, it is preferable to use cut pieces of these materials in order to shorten the time required for dissolution.

Curdlan is an almost pure linear polymer of glucose polymerized by β-1,3-glucosidic bonds. Curdlan is produced from glucose by *Agrobacterium* sp. and is used as food additives, mainly as gelling agents, stabilizers, and thickeners.

Chitin is a polymer of N-acetyl-D-glucosamine polymerized by β-1,4-glycosidic bonds. It is a component of the exoskeleton of arthropods, particularly the shells of crustaceans, the shells of mollusks, particularly shellfish, and the cell walls of fungi (mold) and basidiomycetes (mushrooms), and is the second most abundant natural product after cellulose. Chitin, which is a natural product, is a polysaccharide that contains not only N-acetylglucosamine but also glucosamine as components, and the ratio of N-acetylglucosamine to glucosamine is said to be approximately 9:1.

Chitosan is a deacetylated product of chitin and is a cationic polysaccharide containing a primary amino group.

The polysaccharide-containing liquid composition in the present invention contains a polysaccharide and an onium salt (1) or onium salt composition, and the polysaccharide is dissolved in the onium salt (1) or onium salt composition. A liquid composition composed of a polysaccharide and an onium salt (1) or onium salt composition is referred to as a polysaccharide-containing liquid composition (a1).

Further, the polysaccharide-containing liquid composition (a1) may contain an organic solvent. When an organic solvent is contained, it is referred to as a polysaccharide-containing liquid composition (a2). Examples of the organic solvent include dimethyl sulfoxide, N,N-dimethylacetamide, N,N-dimethylformamide, N-methyl-2-pyrrolidone, acetonitrile, pyridine, methylimidazole, 3-methoxy-2-propanol, hexamethylphosphoric triamide, tetramethylurea, 1,3-dimethyl-2-imidazolidinone, γ-butyrolactone, and the like, but are not particularly limited thereto. Two or more organic solvents may be contained. The polysaccharide-containing liquid composition (a2) is formed by dissolving a polysaccharide in a medium containing the onium salt (1) or onium salt composition, and the organic solvent. The above organic solvent by itself hardly dissolves polysaccharides; however, when mixed with the onium salt (1) or onium salt composition, it can contribute to improving polysaccharide solubility, accelerating the dissolution rate of polysaccharides by reducing viscosity, improving workability in the polysaccharide recovery process, and controlling the physical properties of the recovered polysaccharides.

The ratio of the onium salt (1) or onium salt composition, and the organic solvent in the medium of the polysaccharide-containing liquid composition (a2), {(mass of onium salt (1) or onium salt composition)/[(mass of onium salt (1) or onium salt composition) + mass of organic solvent] × 100}, is generally 0.5 to 99.5 mass%, preferably 10 to 90 mass%, and more preferably 20 to 80 mass%.

The polysaccharide-containing liquid composition (a1) or (a2) may contain a known onium salt other than the onium salt (1). Specific examples of such known onium salts other than the onium salt (1) include 1-ethyl-3-methylimidazolium acetate and the like, but are not particularly limited thereto.

In the polysaccharide-containing liquid composition (a1) or (a2), additives can be used to the extent that they are effective. Examples of additives include flavors, dyes, color pigments, water repellents, oil repellents, antioxidants, hydrolysis inhibitors, flame retardants, ultraviolet light absorbers, light stabilizers, antistatic agents, conductive agents, stress relief agents, mold release agents, crystallization promoters, lubricants, impact agents, sliding property modifiers, nucleating agents, strengthening agents, reinforcing agents, flow regulators, thickeners, antibacterial agents, antifungal agents, rust inhibitors, anti-settling agents, anti-sagging agents, antifoaming agents, coupling agents, fillers, and the like. These additives may be used singly or in combination of two or more.

When the polysaccharide-containing liquid composition (a1) contains not only a polysaccharide and an onium salt (1) or onium salt composition, but also optionally a known onium salt and additives, it is referred to as a polysaccharide-containing liquid composition (a3).

When the polysaccharide-containing liquid composition (a2) contains not only a polysaccharide, an onium salt (1) or onium salt composition, and an organic solvent, but also optionally a known onium salt and additives, it is referred to as a polysaccharide-containing liquid composition (a4).

In the polysaccharide-containing liquid compositions (a1) to (a4), the ratio of the onium salt (1) or onium salt composition in the medium, {(mass of onium salt (1) or onium salt composition)/[(mass of onium salt (1) or onium salt composition) + mass of organic solvent + mass of known onium salt + mass of additives] × 100}, is generally 0.5 to 99.5 mass%, preferably 10 to 90 mass%, and more preferably 20 to 80 mass%.

Next, the polysaccharide dissolution method of the present invention is explained. First, a medium containing an onium salt (1) or onium salt composition, and optionally an organic solvent, a known onium salt, and additives is mixed with a polysaccharide for dissolution to obtain any of polysaccharide-containing liquid compositions (a1) to (a4).

The temperature at which the polysaccharide is dissolved in the medium is not particularly limited as long as it is within the temperature range at which the medium is liquid, and it is sufficient that the liquid is maintained at a specific temperature. The temperature is preferably 100°C or less, and more preferably 10°C or more and 50°C or less.

In the step of dissolving the polysaccharide in the medium, the dissolution of the polysaccharide can be promoted by adding stimulation, such as stirring, shaking, or ultrasonic irradiation, to the mixture of the polysaccharide and the medium.

The content ratio of the polysaccharide in the polysaccharide-containing liquid compositions (a1) to (a4) [{mass of polysaccharide/(polysaccharide-containing liquid compositions (a1) to (a4))} × 100] is not particularly limited, and is generally 0.5 to 99.5 mass%, preferably 1 to 50 mass%, and more preferably 5 to 30 mass%.

When the polysaccharide in the polysaccharide-containing liquid compositions (a1) to (a4) is cellulose, the medium other than the cellulose can dissolve the cellulose under stirring at 25°C preferably with a solubility of 10 mass% or more, more preferably 14 mass% or more, even more preferably 18 mass% or more, and particularly preferably 20 mass% or more.

A higher solubility of cellulose is better; however, the solubility at 25°C under stirring is generally 50 mass% or less, 45 mass% or less, or 40 mass% or less. The solubility can be measured, for example, by using Avicel (registered trademark) PH-101, produced by Sigma-Aldrich Co. LLC, which is microcrystalline cellulose, under stirring for 24 hours.

When the polysaccharide in the polysaccharide-containing liquid compositions (a1) to (a4) is curdlan, the medium other than the curdlan can dissolve the curdlan under stirring at 50°C preferably with a solubility of 10 mass% or more, more preferably 14 mass% or more, even more preferably 18 mass% or more, and particularly preferably 20 mass% or more.

When the polysaccharide in the polysaccharide-containing liquid compositions (a1) to (a4) is chitin, the medium other than the chitin can dissolve the chitin under stirring at 50°C preferably with a solubility of 1 mass% or more, more preferably 2 mass% or more, even more preferably 3 mass% or more, and particularly preferably 5 mass% or more.

In the step of dissolving the polysaccharide in the medium, the order of dissolving the polysaccharide, the onium salt (1), and further optional components, i.e., a known onium salt, an organic solvent, and additives, are not particularly limited. The known onium salt, organic solvent, or additives may be mixed before the polysaccharide is dissolved in the onium salt (1) or onium salt composition, or may be mixed during or after dissolution of the polysaccharide in the onium salt (1) or onium salt composition.

In the present invention, "recovery of the polysaccharide" refers to extracting the polysaccharide or polysaccharide derivative as a composition in a form that is useful for industry from any of the polysaccharide-containing liquid compositions (a1) to (a4). For example, as the form of the polysaccharide, it can be extracted as recycled fibers, nanofibers, films, or the like, but is not particularly limited thereto.

Further, chemical and physical treatments can be performed in the polysaccharide-containing liquid compositions (a1) to (a4) to modify the polysaccharide or cleave the polysaccharide chains to make the recovered compositions functional. These treatments can be performed before or during the recovery of the polysaccharide from the polysaccharide-containing liquid compositions (a1) to (a4). Examples of chemical and physical treatments include oxidation, reduction, pyrolysis, hydrolysis, isomerization, esterification, alkoxylation, acetylation, deacetylation, ion exchange, copolymerization, surface coating, saccharification, shearing, and application of voltage. Examples of the recovered compositions with functionality include, but are not particularly limited to, cellulose-saccharified compositions for biomass fuel production, composite compositions with organic/inorganic materials, and the like.

It is not necessary to completely remove the onium salt (1) or onium salt composition used for dissolution from the recovered polysaccharide or polysaccharide derivative. The recovered composition may be used while the onium salt (1) or onium salt composition remains therein.

Next, the polysaccharide recovery method of the present invention is explained. Any of the polysaccharide-containing liquid compositions (a1) to (a4) obtained by the method described above can be brought into contact with a poor solvent to thereby precipitate the polysaccharide in the mixed solution. The precipitated polysaccharide and the mixed solution are separated by filtration or the like, whereby the polysaccharide can be recovered. The mixed solution from which the polysaccharide is separated contains the onium salt (1) or onium salt composition, and the poor solvent. Further, when a known onium salt, an organic solvent, or additives are used, these are contained (hereinafter the mixed solution from which the polysaccharide is separated is referred to as "the mixed solution").

Examples of the poor solvent used to recover polysaccharides include water, methanol, ethanol, acetone, and a mixed solvent of two or more of them, but are not particularly limited thereto. The amount of poor solvent used is not particularly limited as long as it is an amount sufficient to recover polysaccharides from any of the polysaccharide-containing liquid compositions (a1) to (a4). A person skilled in the art would have been able to determine an appropriate amount to use.

The method for bringing the polysaccharide-containing liquid compositions (a1) to (a4) into contact with a poor solvent is not particularly limited. Examples include a method in which a poor solvent is added dropwise to any of the polysaccharide-containing liquid compositions (a1) to (a4), a method in which any of the polysaccharide-containing liquid compositions (a1) to (a4) is placed in a mold or applied to a sheet, and then immersed in a poor solvent, and a method in which any of the polysaccharide-containing liquid compositions (a1) to (a4) is ejected into a poor solvent.

The onium salt (1) or onium salt composition may be recovered from the mixed solution and reused. For reuse, the recovered onium salt (1) or onium salt composition may contain polysaccharide-derived components. If necessary, the polysaccharide-derived components may be removed from the recovered onium salt (1) or onium salt composition by purification. Examples of the method for recovering the onium salt (1) or onium salt composition from the mixed solution after separation include concentration, liquid separation, recrystallization, dialysis, adsorption, filtration, and the like, but are not particularly limited thereto.

In addition, the organic solvent used for polysaccharide dissolution and the poor solvent can be easily recovered from the mixed solution by distillation, concentration, liquid separation, or the like, and reused.

### Examples

The present invention is described in detail below based on Examples; however, the present invention is not limited thereto.

In Table 1, "Solubility" indicates the maximum mass% of a polysaccharide dissolved in an onium salt [(maximum mass of polysaccharide dissolved in onium salt/mass of onium salt) × 100].

In Table 2, "Onium salt concentration" indicates the mass% of an onium salt to a mixed medium of the onium salt and an organic solvent [(mass of onium salt/mass of mixed medium of onium salt and organic solvent) × 100].

Further, in Table 2, "Solubility" indicates the mass% of a polysaccharide dissolved in a mixed medium of an onium salt and an organic solvent or in the organic solvent [(mass of polysaccharide dissolved in mixed medium of onium salt and organic solvent or in organic solvent/mass of mixed medium of onium salt and organic solvent or organic solvent) × 100].

In the Examples, the dissolved state of the polysaccharide was evaluated using a zoom stereo microscope and a polarized observation lens filter produced by Wraymer Inc. The polysaccharide-containing liquid composition (a1) or (a2) was observed with this microscope, and a state in which there was no undissolved polysaccharide showing birefringence was determined to be a completely dissolved state.

### Example 1-1

260 g (3.22 mol) of a 37 wt% formaldehyde aqueous solution (produced by Tokyo Chemical Industry Co., Ltd.), 290 g (4.83 mol) of acetic acid (produced by Junsei Chemical Co., Ltd.), 573 g (6.43 mol) of 3-methoxypropylamine (produced by Tokyo Chemical Industry Co., Ltd.), and 461 g (3.22 mol) of a 40 wt% glyoxal aqueous solution (produced by Tokyo Chemical Industry Co., Ltd.) were placed in a 5 L three-necked reactor purged with nitrogen. The resulting mixture was heated to 40°C and stirred for 3 hours to synthesize 811 g of 1,3-bis(3-methoxypropyl)imidazolium acetate ([(MeOPr)₂Im][AcO]). The yield was 93%.

The ¹H NMR data results of [(MeOPr)₂Im][AcO] are shown below.
¹H NMR (DMSO-d₆) δ (ppm) = 9.41 (s, 1H), 7.78 (d, 2H), 4.22 (t, 4H), 3.33 (t, 4H), 3.22 (s, 6H), 2.07-2.00 (m, 4H), 1.56 (s, 3H)

### Example 1-2

4.11 g (50.8 mmol) of a 37 wt% formaldehyde aqueous solution (produced by Tokyo Chemical Industry Co., Ltd.), 4.55 g (75.8 mmol) of acetic acid (produced by Junsei Chemical Co., Ltd.), 12.9 g (96.7 mmol) of 3-(2-methoxyethoxy)propylamine (produced by Combi-Blocks), and 7.24 g (50.5 mmol) of a 40 wt% glyoxal aqueous solution (produced by Junsei Chemical Co., Ltd.) were placed in a 100 mL three-necked reactor purged with nitrogen. The resulting mixture was heated to 40°C and stirred for 3 hours to synthesize 13.9 g of 1,3-bis[3-(2-methoxyethoxy)propyl]imidazolium acetate ([(MeOEtOPr)₂Im][AcO]). The yield was 80%.

The ¹H NMR data results of [(MeOEtOPr)₂Im][AcO] are shown below.
¹H NMR (DMSO-d₆) δ (ppm) = 9.42 (s, 1H), 7.80 (d, 2H), 4.23 (t, 4H), 3.49-3.47 (m, 4H), 3.44-3.40 (m, 8H), 3.25 (s, 6H), 2.08-2.01 (m, 4H), 1.52 (s, 3H)

### Example 1-3

40 g of a strongly basic ion exchange resin (produced by FUJIFILM Wako Pure Chemical Corporation, DOWEX (registered trademark) MONOSPHERE 550A (OH)) was mixed with ion exchange water, and the mixture was packed in a column tube. 14.0 g of [(MeOPr)₂Im][AcO] obtained in Example 1-1 was dissolved in ion exchange water to prepare a 0.6 M aqueous solution. The aqueous solution was passed through the column packed with the ion exchange resin, and subsequently 120 g of ion exchange water was passed through the column, thereby obtaining 204 g of a 1,3-bis(3-methoxypropyl) imidazolium hydroxide ([(MeOPr)₂Im]OH) aqueous solution.

35.3 g of a 10 wt% aqueous solution of acrylic acid (produced by Tokyo Chemical Industry Co., Ltd.), which was prepared in advance, was added to the resulting [(MeOPr)₂Im]OH aqueous solution, and the mixture was stirred. After stirring, the resulting reaction liquid was concentrated to obtain 14.3 g of 1,3-bis(3-methoxypropyl)imidazolium acrylate ([(MeOPr)₂Im][Acr]). The yield was 98%.

The ¹H NMR data results of [(MeOPr)₂Im][OH] are shown below.
¹H NMR (D₂O) δ (ppm) = 7.5 (s, 2H), 4.28 (t, 4H), 3.47 (t, 4H), 3.31 (s, 6H), 2.13 (m, 4H)

The ¹H NMR data results of [(MeOPr)₂Im][Acr] are shown below.
¹H NMR (DMSO-d₆) δ (ppm) = 9.72 (s, 1H), 7.83 (d, 2H), 5.93 (dd, 1H), 5.62 (dd, 1H), 5.09 (dd, 1H), 4.25 (t, 4H), 3.33 (t, 4H), 3.21 (s, 6H), 2.08-2.01(m, 4H)

### Example 1-4

1.86 g (23.0 mmol) of a 37 wt% formaldehyde aqueous solution (produced by Tokyo Chemical Industry Co., Ltd.), 2.34 g (38.9 mmol) of acetic acid (produced by Junsei Chemical Co., Ltd.), 3.64 g (48.5 mmol) of 2-methoxyethylamine (produced by Tokyo Chemical Industry Co., Ltd.), and 3.59 g (24.9 mmol) of a 40 wt% glyoxal aqueous solution (produced by Junsei Chemical Co., Ltd.) were placed in a 30 mL three-necked reactor purged with nitrogen. The resulting mixture was heated to 40°C and stirred for 3 hours to synthesize 5.03 g of 1,3-bis(2-methoxyethyl) imidazolium acetate ([(MeOEt)₂Im][AcO]). The yield was 89%. The ¹H NMR data results of [(MeOEt)₂Im][AcO] are shown below.
¹H NMR (DMSO-d₆) δ (ppm) = 9.44 (s, 1H), 7.78 (s, 2H), 4.39 (t, 4H), 3.70 (t, 4H), 3.27 (s, 6H), 1.53 (s, 3H)

### Example 2-1

6.46 g (79.8 mmol) of a 37 wt% formaldehyde aqueous solution (produced by Tokyo Chemical Industry Co., Ltd.), 7.02 g (117 mmol) of acetic acid (produced by Junsei Chemical Co., Ltd.), 16.5 g (160 mmol) of 3-ethoxypropylamine (produced by Tokyo Chemical Industry Co., Ltd.), and 11.8 g (81.8 mmol) of a 40 wt% glyoxal aqueous solution (produced by Junsei Chemical Co., Ltd.) were placed in a 100 mL three-necked reactor purged with nitrogen. The resulting mixture was heated to 40°C and stirred for 3 hours to synthesize 17.7 g of 1,3-bis(3-ethoxypropyl) imidazolium acetate ([(EtOPr)₂Im][AcO]). The yield was 74%.

The ¹H NMR data results of [(EtOPr)₂Im][AcO] are shown below.
¹H NMR (DMSO-d₆) δ (ppm) = 9.97 (s, 1H), 7.86 (d, 2H), 4.25 (t, 4H), 3.39-3.34 (m, 8H), 2.06-2.00 (m, 4H), 1.56 (s, 3H), 1.07 (t, 6H)

### Example 2-2

7.19 g (88.8 mmol) of a 37 wt% formaldehyde aqueous solution (produced by Tokyo Chemical Industry Co., Ltd.), 8.09 g (135 mmol) of acetic acid (produced by Junsei Chemical Co., Ltd.), 8.19 g (91.9 mmol) of 3-methoxypropylamine (produced by Tokyo Chemical Industry Co., Ltd.), 9.17 g (88.8 mmol) of 3-ethoxypropylamine (produced by Kanto Chemical Co., Inc.), and 12.9 g (89.3 mmol) of a 40 wt% glyoxal aqueous solution (produced by Junsei Chemical Co., Ltd.) were placed in a 180 mL three-necked reactor purged with nitrogen. The resulting mixture was heated to 40°C and stirred for 3 hours to synthesize 21.7 g of a mixture of 1-(3-ethoxypropyl)-3-(3-methoxypropyl)imidazolium acetate ([(EtOPr)(MeOPr)Im][AcO]), [(MeOPr)₂Im][AcO], and [(EtOPr)₂Im][AcO] ([(EtOPr/MeOPr)₂Im][AcO]). The yield was 88%.

The ¹H NMR data results of [(EtOPr/MeOPr)₂Im][AcO] are shown below.
¹H NMR (DMSO-d₆) δ (ppm) = 9.75 (s, 1H), 7.82 (d, 2H), 4.24 (t, 4H), 3.40-3.31 (m, 6H), 3.21 (s, 3H), 2.07-2.00 (m, 4H), 1.58 (s, 3H), 1.07 (t, 3H)

### Example 2-3

5.63 g (69.6 mmol) of a 37 wt% formaldehyde aqueous solution (produced by Tokyo Chemical Industry Co., Ltd.), 6.17 g (103 mmol) of acetic acid (produced by Junsei Chemical Co., Ltd.), 17.9 g (136 mmol) of 3-butoxypropylamine (produced by Tokyo Chemical Industry Co., Ltd.), and 9.85 g (68.4 mmol) of a 40 wt% glyoxal aqueous solution (produced by Junsei Chemical Co., Ltd.) were placed in a 180 mL three-necked reactor purged with nitrogen. The resulting mixture was heated to 40°C and stirred for 3 hours to synthesize 17.1 g of 1,3-bis(3-butoxypropyl) imidazolium acetate ([(BuOPr)₂Im][AcO]). The yield was 70%.

The ¹H NMR data results of [(BuOPr)₂Im][AcO] are shown below.
¹H NMR (DMSO-d₆) δ (ppm) = 9.53 (s, 1H), 7.80 (d, 2H), 4.23 (t, 4H), 3.37 (t, 4H), 3.33 (t, 4H), 2.07-2.00 (m, 4H), 1.56 (s, 3H), 1.48-1.41 (m, 4H), 1.33-1.24 (m, 4H), 0.87 (t, 6H)

### Example 2-4

13.0 g of 1,3-bis(3-methoxypropyl)imidazolium propionate ([(MeOPr)₂Im][PrO]) was obtained in the same manner as in Example 1-4, except that 35.3 g of the 10 wt% acrylic acid (produced by Tokyo Chemical Industry Co., Ltd.) aqueous solution in Example 1-3 was changed to 36.9 g of a 10 wt% propionic acid (produced by Junsei Chemical Co., Ltd.) aqueous solution. The yield was 87%.
¹H NMR (CDCl₃) δ (ppm) = 11.91 (s, 1H), 7.10 (d, 2H), 4.43 (t, 4H), 3.39 (t, 4H), 3.29 (t, 6H), 2.26-2.20 (m, 2H), 2.19-2.12 (m, 4H), 1.15-1.10 (m, 3H)

### Example 2-5

15.0 g of 1,3-bis(3-methoxypropyl)imidazolium methacrylate ([(MeOPr)₂Im][MeAcr]) was obtained in the same manner as in Example 1-4, except that 35.3 g of the 10 wt% acrylic acid (produced by Tokyo Chemical Industry Co., Ltd.) aqueous solution in Example 1-3 was changed to 42.4 g of a 10 wt% methacrylic acid (produced by Tokyo Chemical Industry Co., Ltd.) aqueous solution. The yield was 97%.

The ¹H NMR data results of [(MeOPr)₂Im][MeAcr] are shown below.
¹H NMR (DMSO-d₆) δ (ppm) = 9.72 (s, 1H), 7.83 (d, 2H), 5.43-5.42 (m, 1H), 4.85-4.83 (m, 1H), 4.25 (t, 4H), 3.33 (t, 4H), 3.22 (s, 6H), 2.08-2.01 (m, 4H), 1.73 (dd, 3H)

### Example 2-6

6.70 g (82.8 mmol) of a 37 wt% formaldehyde aqueous solution (produced by Tokyo Chemical Industry Co., Ltd.), 7.64 g (127 mmol) of acetic acid (produced by Junsei Chemical Co., Ltd.), 17.3 g (165 mmol) of 3-methylthiopropylamine (produced by Tokyo Chemical Industry Co., Ltd.), and 12.3 g (85.5 mmol) of a 40 wt% glyoxal aqueous solution (produced by Junsei Chemical Co., Ltd.) were placed in a 100 mL three-necked reactor purged with nitrogen. The resulting mixture was heated to 40°C and stirred for 3 hours to synthesize 19.3 g of 1,3-bis(3-methylthiopropyl) imidazolium acetate ([(MeSPr)₂Im][AcO]). The yield was 77%.

The ¹H NMR data results of [(MeSPr)₂Im][AcO] are shown below.
¹H NMR (DMSO-d₆) δ (ppm) = 9.93 (s, 1H), 7.87 (d, 2H), 4.27 (t, 4H), 2.46 (t, 4H), 2.09-2.04 (m, 4H), 2.04 (s, 6H), 1.59 (s, 3H)

### Evaluation Example 1-1

1.00 g of [(MeOPr)₂Im][AcO] was placed in a sample tube and kept at 25°C.

Microcrystalline cellulose (Avicel (registered trademark) PH-101, produced by Sigma-Aldrich Co. LLC) was added in 0.01 g increments at 25°C and stirred. This operation was repeated until the microcrystalline cellulose was no longer completely dissolved after 10 minutes to 24 hours. The results of solubility are shown in Table 1.

### Evaluation Examples 1-2, 1-3, and 2-1 to 2-3, and Comparative Example 1-1

The same operation was performed as in Evaluation Example 1-1, except that the onium salt of Evaluation Example 1-1 was changed to the onium salts listed in Table 1. The results of solubility are shown in Table 1. The 1-ethyl-3-methylimidazolium acetate used in Comparative Example 1-1 was the one produced by Tokyo Chemical Industry Co., Ltd.

**Table 1**

| | Onium salt | Solubility (mass%) |
|---|---|---|
| Evaluation Example 1-1 | [(MeOPr)₂Im][AcO] | 25 |
| Evaluation Example 1-2 | [(MeOEtOPr)₂Im][AcO] | 20 |
| Evaluation Example 1-3 | [(MeOPr)₂Im][Acr] | 19 |
| Evaluation Example 2-1 | [(EtOPr) ₂Im][AcO] | 10 |
| Evaluation Example 2-2 | [(EtOPr/MeOPr)₂Im][AcO] | 21 |
| Evaluation Example 2-3 | [(MeOPr)₂Im][PrO] | 19 |
| Comparative Example 1-1 | 1-Ethyl-3-methylimidazolium acetate | 3 |

The onium salts shown in Evaluation Examples 1-1 to 1-3 and 2-1 to 2-3 showed superior cellulose solubility at 25°C compared to the onium salt shown in Comparative Example 1-1.

### Evaluation Example 1-4

0.63 g of [(MeOPr)₂Im][AcO] and 0.44 g of dimethyl sulfoxide (hereinafter abbreviated as DMSO) were placed in a sample tube to prepare a mixture. The same operation was performed as in Evaluation Example 1-1, except that the onium salt of Evaluation Example 1-1 was changed to 1.0 g of the mixture. The results of solubility are shown in Table 2.

### Evaluation Examples 1-5 and 2-4 to 2-6

The same operation was performed as in Evaluation Example 1-4, except that the onium salt of Evaluation Example 1-4 was changed to the onium salts listed in Table 2. The results of solubility are shown in Table 2.

### Comparative Example 1-2

The same operation was performed as in Evaluation Example 1-4, except that the mixture of Evaluation Example 1-4 was changed to DMSO. The results of solubility are shown in Table 2.

**Table 2**

| | Mixture | | Onium salt concentration (mass%) | Solubility (mass%) |
|---|---|---|---|---|
| | Onium salt | Organic solvent | | |
| Evaluation Example 1-4 | [(MeOPr)₂Im][AcO] | DMSO | 59 | 22 |
| Evaluation Example 1-5 | [(MeOEt)₂Im][AcO] | DMSO | 59 | 21 |
| Evaluation Example 2-4 | [(BuOPr)₂Im][AcO] | DMSO | 59 | 6 |
| Evaluation Example 2-5 | [(MeOPr)₂Im][MeAcr] | DMSO | 59 | 15 |
| Evaluation Example 2-6 | [(MeSPr)₂Im][AcO] | DMSO | 59 | 16 |
| Comparative Example 1-2 | None | DMSO | 0 | 1 or less |

### Evaluation Example 2-7

1.00 g of [(MeOPr)₂Im][AcO] was placed in a sample tube and kept at 50°C.

Curdlan (produced by FUJIFILM Wako Pure Chemical Corporation) was added in 0.01 g increments at 50°C and stirred. This operation was repeated until curdlan was no longer completely dissolved after 10 minutes to 24 hours. The results of solubility are shown in Table 3.

**Table 3**

| | Onium salt | Solubility (mass%) |
|---|---|---|
| Evaluation Example 2-7 | [(MeOPr)₂Im][AcO] | 29 |

The onium salt shown in Evaluation Example 2-7 showed excellent curdlan solubility at 50°C.

### Evaluation Example 2-8

1.00 g of [(MeOPr)₂Im][AcO] was placed in a sample tube and kept at 50°C.

Chitin (produced by FUJIFILM Wako Pure Chemical Corporation) was added in 0.01 g increments at 50°C and stirred. This operation was repeated until chitin was no longer completely dissolved after 10 minutes to 24 hours. The results of solubility are shown in Table 4.

### Comparative Example 2-1

The same operation was performed as in Evaluation Example 2-8, except that the onium salt of Evaluation Example 2-8 was changed to the onium salt listed in Table 4. The results of solubility are shown in Table 4.

**Table 4**

| | Onium salt | Solubility (mass%) |
|---|---|---|
| Evaluation Example 2-8 | [(MeOPr)₂Im][AcO] | 6 |
| Comparative Example 2-1 | 1-Ethyl-3-methylimidazolium acetate | 1 or less |

The onium salt shown in Evaluation Example 2-8 showed superior chitin solubility at 50°C compared to the onium salt shown in Comparative Example 2-1.

### Evaluation Example 1-6

2.00 g of [(MeOPr)₂Im][AcO] and 0.20 g of microcrystalline cellulose were placed in a sample tube and stirred at 25°C to prepare a cellulose-containing liquid composition. The cellulose-containing liquid composition was placed in a plastic syringe and extruded into a water bath at 15 to 20°C, followed by washing and drying, whereby it was confirmed that fibrous cellulose could be recovered.

### Evaluation Example 2-9

10.00 g of [(MeOPr)₂Im][AcO] and 2.00 g of microcrystalline cellulose were placed in a sample tube and stirred at 25°C to prepare a cellulose-containing liquid composition. The cellulose-containing liquid composition was applied to a glass plate and spread with an applicator on a hot plate heated to 60°C. The spread cellulose-containing liquid composition, together with the glass plate, was led into a water bath at 15 to 20°C, followed by washing and drying, whereby it was confirmed that the film-like cellulose could be recovered.

### Industrial Applicability

According to the present invention, it is possible to provide an onium salt with very high polysaccharide solubility at 10 to 50°C, particularly at a temperature around room temperature (25°C). It is also possible to provide a liquid composition comprising the onium salt and polysaccharides as a composition suitable for recovering polysaccharides, and a method for recovering polysaccharides efficiently using such a liquid composition comprising the onium salt and polysaccharides.

## Claims

1. An onium salt represented by the following Formula (1): 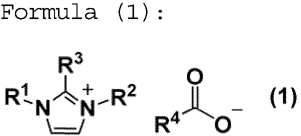
wherein R¹ and R² are the same or different, and each represents a C₂-C₁₂ hydrocarbon group containing 1 to 5 heteroatoms or a group represented by the following Formula (1a):
-O-R^{a} (1a)
wherein R² represents a C₁-C₁₂ hydrocarbon group that may contain 1 to 4 heteroatoms;
R³ represents a hydrogen atom or a hydrocarbon group that may contain a heteroatom, wherein the heteroatom is at least one member selected from the group consisting of nitrogen, oxygen, and sulfur atoms; and
R⁴ represents a hydrocarbon group, provided that when R¹ and R² are methoxyethyl groups and R³ is a hydrogen atom, R⁴ represents a C₂ or more hydrocarbon group.

2. The onium salt according to claim 1, wherein R³ in Formula (1) is a hydrogen atom or a methyl group.

3. The onium salt according to claim 1 or 2, wherein R¹ and R² in Formula (1) are the same or different, and each is represented by Formula (2):
Formula (2):
-R⁵-X-R⁶ (2)
wherein R⁵ represents a C₂-C₅ divalent hydrocarbon group, R⁶ represents a C₁-C₁₀ hydrocarbon group that may contain 1 to 4 heteroatoms, the total number of carbon atoms in R⁵ and R⁶ is 3 to 12, the heteroatom is at least one member selected from the group consisting of nitrogen, oxygen, and sulfur atoms, and X represents an oxygen atom or a sulfur atom.

4. The onium salt according to claim 3, wherein in Formula (2), R⁵ is a propane-1,3-diyl group, R⁶ is a C₁-C₉ hydrocarbon group that may contain 1 to 4 heteroatoms, and X is an oxygen atom.

5. The onium salt according to claim 1, wherein R¹ and R² in Formula (1) are the same.

6. The onium salt according to claim 1, wherein R⁴ in Formula (1) is a methyl group, CH₂=CH-, or CH₂=C(CH₃)-.

7. An onium salt composition comprising two or more onium salts represented by the following Formula (1): 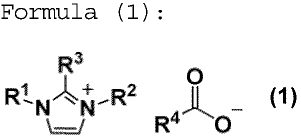
wherein R¹ and R² are the same or different, and each represents a C₂-C₁₂ hydrocarbon group containing 1 to 5 heteroatoms or a group represented by the following Formula (1a):
-O-R^{a} (1a)
wherein R^{a} represents a C₁-C₁₂ hydrocarbon group that may contain 1 to 4 heteroatoms;
R³ represents a hydrogen atom or a hydrocarbon group that may contain a heteroatom, wherein the heteroatom is at least one member selected from the group consisting of nitrogen, oxygen, and sulfur atoms; and
R⁴ represents a hydrocarbon group.

8. An onium hydroxide salt represented by the following Formula (3): 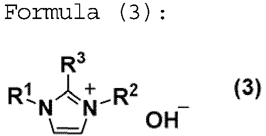
wherein R¹ and R² are the same or different, and each represents a C₂-C₁₂ hydrocarbon group containing 1 to 5 heteroatoms or a group represented by the following Formula (1a):
-O-R^{a} (1a)
wherein R² represents a C₁-C₁₂ hydrocarbon group that may contain 1 to 4 heteroatoms; and
R³ represents a hydrogen atom or a hydrocarbon group that may contain a heteroatom, wherein the heteroatom is at least one member selected from the group consisting of nitrogen, oxygen, and sulfur atoms.

9. A method for producing an onium salt represented by Formula (1), comprising reacting glyoxal, an amine compound represented by Formula (6a), an amine compound represented by Formula (6b), an aldehyde compound represented by Formula (7), and a carboxylic acid represented by Formula (4):
Formula (6a):
R¹-NH₂ (6a)
Formula (6b):
R²-NH₂ (6b)
wherein in Formulas (6a) and (6b), R¹ and R² are the same or different, and each represents a C₂-C₁₂ hydrocarbon group containing 1 to 5 heteroatoms or a group represented by the following Formula (1a);
-O-R^{a} (1a)
wherein R^{a} represents a C₁-C₁₂ hydrocarbon group that may contain 1 to 4 heteroatoms;
Formula (7):
R³-CHO (7)
wherein R³ represents a hydrogen atom or a hydrocarbon group that may contain a heteroatom, wherein the heteroatom is at least one member selected from the group consisting of nitrogen, oxygen, and sulfur atoms;
Formula (4):
R⁴-COOH (4)
wherein R⁴ represents a hydrocarbon group; and
Formula (1): 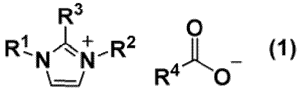
wherein R¹ to R⁴ are as defined above.

10. A method for producing an onium salt represented by Formula (1), comprising exchanging ions in a compound represented by Formula (3) using a compound represented by Formula (4): 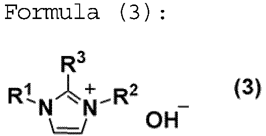
wherein R¹ and R² are the same or different, and each represents a C₂-C₁₂ hydrocarbon group containing 1 to 5 heteroatoms or a group represented by the following Formula (1a):
-O-R^{a} (1a)
wherein R^{a} represents a C₁-C₁₂ hydrocarbon group that may contain 1 to 4 heteroatoms;
R³ represents a hydrogen atom or a hydrocarbon group that may contain a heteroatom, wherein the heteroatom is at least one member selected from the group consisting of nitrogen, oxygen, and sulfur atoms;
Formula (4):
R⁴-COOH (4)
wherein R⁴ represents a hydrocarbon group; and
Formula (1): 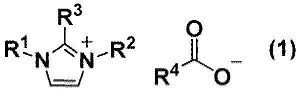
wherein R¹ to R⁴ are as defined above.

11. A polysaccharide-containing liquid composition comprising at least one polysaccharide and an onium salt represented by the following Formula (1) or the onium salt composition according to claim 7, wherein the polysaccharide is dissolved in the onium salt: 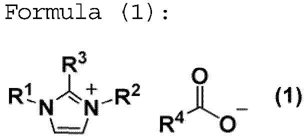
wherein R¹ and R² are the same or different, and each represents a C₂-C₁₂ hydrocarbon group containing 1 to 5 heteroatoms or a group represented by the following Formula (1a):
-O-R^{a} (1a)
wherein R^{a} represents a C₁-C₁₂ hydrocarbon group that may contain 1 to 4 heteroatoms;
R³ represents a hydrogen atom or a hydrocarbon group that may contain a heteroatom, wherein the heteroatom is at least one member selected from the group consisting of nitrogen, oxygen, and sulfur atoms; and
R⁴ represents a hydrocarbon group, provided that when R¹ is (2-methoxyethoxy)methyl, R² is 2-[2-(2-methoxyethoxy)ethoxy]ethyl, and R³ is a hydrogen atom, R⁴ represents a C₂ or more hydrocarbon group.

12. The polysaccharide-containing liquid composition according to claim 11, further comprising at least one organic solvent, wherein the polysaccharide is dissolved in a medium containing the onium salt or onium salt composition and the organic solvent.

13. The polysaccharide-containing liquid composition according to claim 12, wherein the organic solvent is at least one organic solvent selected from the group consisting of dimethyl sulfoxide, N,N-dimethylacetamide, N,N-dimethylformamide, and N-methyl-2-pyrrolidone.

14. The polysaccharide-containing liquid composition according to claim 11, wherein the polysaccharide is cellulose.

15. A method for producing the polysaccharide-containing liquid composition according to claim 11, comprising dissolving a polysaccharide using an onium salt represented by the following Formula (1) or the onium salt composition according to claim 7:
Formula (1): 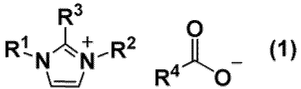
wherein R¹ and R² are the same or different, and each represents a C₂-C₁₂ hydrocarbon group containing 1 to 5 heteroatoms or a group represented by the following Formula (1a):
-O-R^{a} (1a)
wherein R^{a} represents a C₁-C₁₂ hydrocarbon group that may contain 1 to 4 heteroatoms;
R³ represents a hydrogen atom or a hydrocarbon group that may contain a heteroatom, wherein the heteroatom is at least one member selected from the group consisting of nitrogen, oxygen, and sulfur atoms; and
R⁴ represents a hydrocarbon group.

16. The method for producing the polysaccharide-containing liquid composition according to claim 15, wherein the temperature to dissolve the polysaccharide is 50°C or less.

17. The method for producing the polysaccharide-containing liquid composition according to claim 15 or 16, wherein the polysaccharide is cellulose.

18. A polysaccharide recovery method, comprising bringing the polysaccharide-containing liquid composition according to any one of claims 11 to 14 into contact with at least one poor solvent.

19. The polysaccharide recovery method according to claim 18, wherein the poor solvent is selected from the group consisting of water, methanol, ethanol, acetone, and a mixed solvent thereof.

20. The polysaccharide recovery method according to claim 18, wherein the polysaccharide is cellulose.
